# EUROPEAN PATENT APPLICATION

(11) **EP 4 617 282 A1**
(43) Date of publication of application: **17.09.2025**
(21) Application number: 23888113.0
(22) Date of filing: 10.11.2023
(51) Int. Cl.: C07K 14/155, C07K 1/06, C07K 1/107, A61K 38/16, A61P 31/12

(54) **MEMBRANE FUSION INHIBITOR FOR INHIBITING AIDS VIRUS AND DRUG-RESISTANT STRAIN THEREOF, AND PHARMACEUTICAL USE THEREOF**

(30) Priority: 11.11.2022 CN 202211410040
(71) Applicant: Institute Of Pathogen Biology, Chinese Academy Of Medical Sciences, Dong Cheng District Beijing 100730 (CN)
(72) Inventor: HE, Yuxian, Beijing 100730 (CN); ZHU, Yuanmei, Beijing 100730 (CN); GENG, Xiuzhu, Beijing 100730 (CN); CHONG, Huihui, Beijing 100730 (CN)
(74) Representative: Ipsilon
(86) International application number: PCT/CN2023/130970
(87) International publication number: WO 2024/099428

(57) **Abstract**

Provided are a membrane fusion inhibitor for inhibiting HIV and a drug-resistant strain thereof, a derivative thereof, a pharmaceutical composition thereof, and a pharmaceutical use thereof.

## Description

The present application is based on and claims the benefit of priority from Chinese Application No. 202211410040.4, filed on Nov. 11, 2022, the disclosures of which are incorporated herein by reference in its entirety.

### Technical Field

The present application belongs to the biomedicine field, and relates to a membrane fusion inhibitor for inhibiting a human immunodeficiency virus and a drug-resistant strain thereof, a derivative thereof, or a pharmaceutical composition thereof, and to a pharmaceutical use thereof.

### Background Art

Acquired Immune Deficiency Syndrome (AIDS) is caused by human immunodeficiency virus (HIV) infection, and at present, there are about 38.4 million of people who are infected in the world (www.unaids.org). Since HIV vaccines have not been successfully developed, drugs that block the replication of the virus at different stages play a major role in the treatment and prevention of HIV infection. Drugs used in clinical treatment mainly include nucleoside reverse transcriptase inhibitors, non-nucleoside reverse transcriptase inhibitors, protease inhibitors, viral entry inhibitors, and integrase inhibitors (www.fda.gov). A highly effective antiviral treatment regimen ("cocktail" therapy) that has been widely used consists mainly of three to four reverse transcriptase inhibitors and protease inhibitors. Due to the persistence of HIV infection, it is required to administer drugs to patients for a long period of time, easily leading to drug resistance, which seriously affects the clinical treatment effect. Accordingly, the development of new anti-HIV drugs has always been an important demand for preventing and controlling AIDS.

Entry of HIV into target cells is mediated by its surface envelope glycoprotein (Env), which is formed by binding a surface subunit gp120 to a transmembrane subunit gp41 via a non-covalent bond. During HIV infection, the sequential binding of gp120 to the cellular receptor CD4 receptor and co-receptor (such as CCR5 or CXCR4) triggers conformational changes in gp120, further exposes the gp41 and activates the membrane fusion function of the gp41. The N-terminal fusion peptide (FP) of the gp41 is first inserted into the membrane of a target cell, then the C-terminal heptad region (CHR) is reversely bound to the N-terminal heptad region (NHR) to form a stable six-helix bundle (6-HB) structure, which brings the viral membrane close to the cell membrane for fusion, whereby the HIV genetic material enters into the cell to result in infections. The structure shows that the C-terminus of the NHR helix contains a hydrophobic deep pocket, while the sequence (WMEWDREI) at the N-terminus of the CHR helix is a pocket-binding domain (PBD), in which evolutionarily highly conserved two tryptophan (W) and one isoleucine (I) are inserted into the NHR pocket to mediate extensive hydrophobic effects, which plays an important role in the formation of the 6-HB structure and in the virus infection (Chan et al., 1997). The NHR hydrophobic pocket has been always recognized as an important drug target.

HIV membrane fusion inhibitors function at the early stages of virus replication, and act by blocking the virus from entering the target cells, and thus they have obvious advantages in both treatment and prevention. However, only enfuvirtide (also known as T20) is currently approved by U.S Food and Drug Administration (FDA) for clinical application. The T20 is a polypeptide having 36 amino acids derived from the CHR of the virus and free of the PBD sequence, and it exerts its antiviral effect by competitively binding to the NHR to block the formation of the 6-HB of the virus. The T20, due to its relatively low activity for inhibiting virus and its relatively short half-life, is required to be administered at a large dose every day, and it easily leads to drug resistance, thereby widely limiting its clinical applications. Hence, the development of new HIV membrane fusion inhibitors is always concerned at home and abroad.

At present, the research and development of HIV membrane fusion inhibitors are mostly focused on the effects of PBD, especially on the C34 polypeptide published in the early years as a design template (He, 2013). The C34 contains PBD and the amino acid sequence of the C34 is recognized as the core sequence of the CHR. Both Sifuvirtide (SFT) and Albuvirtide (ABT), which are early developed in China, are designed based on the C34 sequence. The SFT is obtained by mutating 14 amino acids of the C34, and adding serine (S) and glutamic acid (E) at N-terminus and C-terminus, respectively, to increase the stability and target binding ability of the polypeptide. The ABT is obtained by only mutating three amino acids of the C34, and incorporating 3-maleimidopropanoic acid (MPA) to the lysine (K) side chain at position 13 to make it has the ability for binding serum albumin. Although the antiviral activities of both SFT and ABT are not substantially improved in comparison to T20 and C34, they have prolonged biological half-life, especially ABT, which can achieve the therapeutic effect of once administration every week.

The present inventor team has long been engaged in research and development of HIV membrane fusion inhibitors, and has designed a plurality of CHR polypeptide-based membrane fusion inhibitors according to the structure and function relationship of the gp41 (Xue et al, 2022). Among them, LP-98, like T20, does not contain the PBD sequence in its molecular structure, and by removing 8 amino acids at the C-terminus of T20, and mutating 16 amino acids in the retained 28 amino acids to promote E and K amino acids to form a "salt bridge structure", the stability of the helical structure of the polypeptide is remarkably improved. Meanwhile, the LP-98 becomes into a lipopeptide by modifying the side chain of K at the C-terminus by cholesterol. Research shows that the average IC₅₀ value of the LP-98 inhibiting a group of replicative HIV strains from infecting different target cells reaches an extremely low picomolar (pM) level, which is increased by 273368 folds in comparison to the T20, and increased by 120789 folds and 376368 folds in comparison to first-list AIDS drugs Zidovudine (AZT) and Lamivudine (3TC), respectively. The activity of LP-98 in inhibiting 36 representative international epidemic HIV-1 subtype pseudoviruses is increased by 10504 folds in comparison to the T20 and by 3751 folds in comparison to the AZT; the LP-98 has extremely strong therapeutic and prophylactic effects when administered to a monkey infection model at a low dose (Xue et al, 2022). Nevertheless, the inventors also find that the LP-98 has a significantly reduced inhibitory activity against T20 resistant HIV strains so that its druggability is affected.

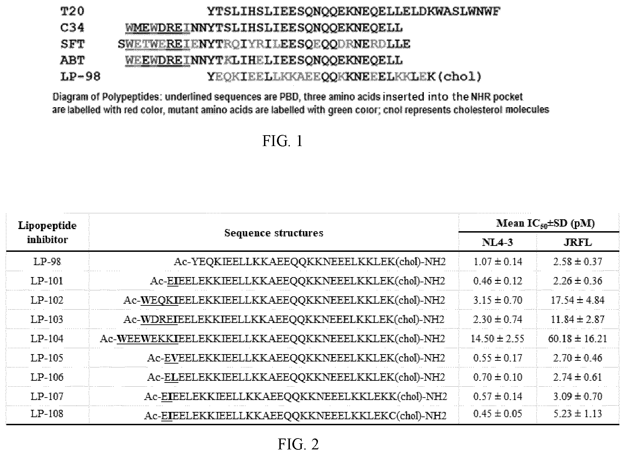

A diagram for sequence comparisons of T20, C34, SFT, ABT and LP-98 is shown in FIG. 1.

The references:
1. Chan, D.C., Fass, D., Berger, J.M., Kim, P.S., 1997. Core structure of gp41 from the HIV envelope glycoprotein. Cell 89, 263-273.
2. Chong, H., Yao, X., Zhang, C., Cai, L., Cui, S., Wang, Y., He, Y., 2012. Biophysical property and broad anti-HIV activity of albuvirtide, a 3-maleimimidopropionic acid-modified peptide fusion inhibitor. PloS one 7, e32599.
3. Dwyer, J.J., Wilson, K.L., Davison, D.K., Freel, S.A., Seedorff, J.E., Wring, S.A., Tvermoes, N.A., Matthews, T.J., Greenberg, M.L., Delmedico, M.K., 2007. Design of helical, oligomeric HIV-1 fusion inhibitor peptides with potent activity against enfuvirtide-resistant virus. Proceedings of the National Academy of Sciences of the United States of America 104, 12772-12777.
4. He, Y., 2013. Synthesized peptide inhibitors of HIV-1 gp41-dependent membrane fusion. Curr Pharm Des 19, 1800-1809.
5. Hu, Y, Yu, W., Geng, X., Zhu, Y., Chong, H., He, Y., 2022. In Vitro Selection and Characterization of HIV-1 Variants with Increased Resistance to LP-40, Enfuvirtide-Based Lipopeptide Inhibitor. International journal of molecular sciences 23.
6. Su, Y., Chong, H., Qiu, Z., Xiong, S., He, Y., 2015a. Mechanism of HIV-1 Resistance to Short-Peptide Fusion Inhibitors Targeting the Gp41 Pocket. Journal of virology 89, 5801-5811.
7. Su, Y., Chong, H., Xiong, S., Qiao, Y., Qiu, Z., He, Y., 2015b. Genetic Pathway of HIV-1 Resistance to Novel Fusion Inhibitors Targeting the Gp41 Pocket. Journal of virology 89, 12467-12479.
8. Wu, X., Liu, Z., Ding, X., Yu, D., Wei, H., Qin, B., Zhu, Y., Chong, H., Cui, S., He, Y., 2018. Mechanism of HIV-1 Resistance to an Electronically Constrained alpha-Helical Peptide Membrane Fusion Inhibitor. Journal of virology 92, e02044-02017.
9. Xue, J., Chong, H., Zhu, Y., Zhang, J., Tong, L., Lu, J., Chen, T., Cong, Z., Wei, Q., He, Y., 2022. Efficient treatment and pre-exposure prophylaxis in rhesus macaques by an HIV fusion-inhibitory lipopeptide. Cell 185, 131-144 e118.
10. Yu, D., Ding, X., Liu, Z., Wu, X., Zhu, Y., Wei, H., Chong, H., Cui, S., He, Y., 2018. Molecular mechanism of HIV-1 resistance to sifuvirtide, a clinical trial-approved membrane fusion inhibitor. The Journal of biological chemistry 293, 12703-12718.
11. Yu, D., Xue, J., Wei, H., Cong, Z., Chen, T., Zhu, Y., Chong, H., Wei, Q., Qin, C., He, Y., 2020. Therapeutic Efficacy and Resistance Selection of a Lipopeptide Fusion Inhibitor in Simian Immunodeficiency Virus-Infected Rhesus Macaques. Journal of virology 94, e00384-00320.

### Summary

The technical problem to be solved by the present application is how to potently inhibit HIV and drug-resistant strains thereof. The present application is devoted to developing a super-potent, broad-spectrum and long-acting HIV membrane fusion inhibitor with a novel structure, with the expectation of reflecting great clinical development values and application prospects. Accordingly, the present application provides a membrane fusion inhibitor for inhibiting HIV and a drug-resistant strain thereof, a derivative thereof, a pharmaceutical composition thereof and a pharmaceutical use thereof.

The present application provides a lipopeptide, a pharmaceutically acceptable salt thereof, a solvate thereof, a hydrate thereof, or a derivative thereof.

As one form, the lipopeptide is a compound represented by Formula I;

Formula I: X₁-polypeptide P1-X₂;

the polypeptide P1 is the following (a1) or (a2) or (a3):
(a1) a polypeptide with an amino acid sequence "EXEELEKKIEELLKKAEEQQKKNEEELKKLEK";
(a2) a polypeptide with antiviral activity obtained by substitution and/or deletion and/or addition of amino acid residue on the basis of (a1);
(a3) a polypeptide with a sequence identity of at least 60%, 65%, 70%, 75%, 80%, 85%, 90%, 93%, 94%, 95%, 96%, 97%, 98%, or 99% as compared with (a1) and having antiviral activity;
the terminal amino acid residue of the polypeptide P1 is modified with a lipophilic compound group;
X₁ is an amino-terminal protecting group of the polypeptide P1;
X₂ is a carboxyl-terminal protecting group of the polypeptide P1.

Specifically, in the polypeptide P1, X is isoleucine (I), valine (V) or leucine (L).

It is well known in the art that: the amino acids at the 2^{nd} position (X), the 9^{th} position (I), the 16^{th} position (A), the 23^{rd} position (N) and the 30^{th} position (L) in (a1) correspond to the amino acids at the position a of the CHR helix, the amino acids at the 5^{th} position (L), the 12^{th} position (L), the 19^{th} position (Q) and the 26^{th} position (E) in (a1) correspond to the amino acids at the position d of the CHR helix, and these amino acids at the position a and at the position d are key amino acids for the formation of the 6-HB structure by binding the CHR with the NHR target sequence, and they are conservative in sequence and function so that they are not readily substituted by other amino acids. In contrast, the amino acids corresponding to other positions (positions b, c, e, f and g) of the CHR helix have no or little direct interaction with the NHR and are therefore readily substituted by other amino acids without affecting or with only slightly affecting the antiviral activity of the polypeptide.

Accordingly, the substitution and/or deletion and/or addition are located at positions other than the 2^{nd} position (X), the 9^{th} position (I), the 16^{th} position (A), the 23^{rd} position (N), the 30^{th} position (L), the 5^{th} position (L), the 12^{th} position (L), the 19^{th} position (Q), and the 26^{th} position (E) in (a1).

As another form, the lipopeptide is a compound represented by Formula II;

Formula II: X₃-polypeptide P2-X₄;

the polypeptide P2 is the following (b1) or (b2) or (b3):
(b1) a polypeptide with an amino acid sequence "EXEELEKKIEELLKKAEEQQKKNEEELKKLEKX";
(b2) a polypeptide with antiviral activity obtained by substitution and/or deletion and/or addition of amino acid residue on the basis of (b1);
(b3) a polypeptide with a sequence identity of at least 60%, 65%, 70%, 75%, 80%, 85%, 90%, 93%, 94%, 95%, 96%, 97%, 98%, or 99% as compared with (b1) and having antiviral activity;
the terminal amino acid residue of the polypeptide P2 is modified with a lipophilic compound group;
X₃ is an amino-terminal protecting group of the polypeptide P2;
X₄ is a carboxyl-terminal protecting group of the polypeptide P2.

Specifically, in the polypeptide P2, "X" as the 2^{nd} amino acid residue is isoleucine (I), valine (V), or leucine (L), and "X" as the 33^{rd} amino acid residue is lysine (K) or cysteine (C). In some embodiments, in the polypeptide P2, "X" as the 2^{nd} amino acid residue is isoleucine (I), and "X" as the 33^{rd} amino acid residue is lysine (K) or cysteine (C). In some embodiments, in the polypeptide P2, "X" as the 2^{nd} amino acid residue is valine (V). In some embodiments, in the polypeptide P2, "X" as the 2^{nd} amino acid residue is leucine (L). In some embodiments, in the polypeptide P2, "X" as the 33^{rd} amino acid residue is cysteine (C). In some embodiments, in the polypeptide P2, "X" as the 33^{rd} amino acid residue is lysine (K).
It is well known in the art that: the amino acids at the 2^{nd} position (X), the 9^{th} position (I), the 16^{th} position (A), the 23^{rd} position (N) and the 30^{th} position (L) in (b1) correspond to the amino acids at the position a of the CHR helix, the amino acids at the 5^{ffi} position (L), the 12^{th} position (L), the 19^{th} position (Q) and the 26^{th} position (E) in (b1) correspond to the amino acid at the position d of the CHR helix, and these amino acids at the position a and at the position d are key amino acids for the formation of the 6-HB structure by binding the CHR with the NHR target sequence, and they are conservative in sequence and function so that they are not readily substituted by other amino acids. In contrast, the amino acids corresponding to other positions (positions b, c, e, f and g) of the CHR helix have no or little direct interaction with the NHR and are therefore readily substituted by other amino acids without affecting or with only slightly affecting the antiviral activity of the polypeptide.

Accordingly, the substitution and/or deletion and/or addition are located at positions other than the 2^{nd} position (X), the 9^{th} position (I), the 16^{th} position (A), the 23^{rd} position (N), the 30^{th} position (L), the 5^{th} position (L), the 12^{th} position (L), the 19^{th} position (Q), and the 26^{th} position (E) in (b1).

The lipophilic compound is a lipid compound such as cholesterol, a cholesterol derivative (such as cholesteryl hemisuccinate, 2-cholesteryl acetic acid, 2-cholesteryl propionic acid, 3-cholesteryl propionic acid, 2-cholesteryl butyric acid, 2-cholesteryl isobutyric acid, 3-cholesteryl butyric acid, 3-cholesteryl isobutyric acid, 4-cholesteryl butyric acid, 2-cholesteryl valeric acid, 2-cholesteryl isovaleric acid, 3-cholesteryl valeric acid, 5-cholesteryl valeric acid, 2-cholesteryl caproic acid, 6-cholesteryl caproic acid, 2-cholesteryl enanthic acid, 7-cholesteryl enanthic acid, 2-cholesteryl caprylic acid, 8-cholesteryl caprylic acid, and cholesteryl bromoacetate), a fatty acid containing 8 to 20 carbon atoms (such as octadecanoic acid or palmitic acid), dihydrosphingosine (DHS) or vitamin E.

Specifically, the lipophilic compound is cholesterol. In some embodiments, the lipophilic compound is cholesteryl hemisuccinate. In some embodiments, the lipophilic compound is cholesteryl bromoacetate. In some embodiments, the lipophilic compound is octadecanoic acid (stearic acid). In some embodiments, the lipophilic compound is palmitic acid. In some embodiments, the lipophilic compound is dihydrosphingosine. In some embodiments, the lipophilic compound is vitamin E.

When the terminal amino acid residue of the polypeptide is lysine, specifically, the cholesterol modification of the terminal amino acid residue is achieved by performing an amidation reaction of the side chain amino group of the C-terminal lysine of the peptide chain.

When the terminal amino acid residue of a polypeptide is cysteine, specifically, the cholesterol modification of the terminal amino acid residue is achieved by performing a thioether-forming reaction between the side chain sulfhydryl group of the C-terminal cysteine of the peptide chain and cholesteryl bromoacetate.

When the terminal amino acid residue of a polypeptide is lysine, specifically, the stearic acid modification of the terminal amino acid residue is achieved by performing an amidation reaction of the side chain amino group of the C-terminal lysine of the peptide chain.

In some embodiments, the cholesterol is cholesteryl hemisuccinate, which achieves modification to the polypeptide by performing an amidation reaction with the side chain amino group of the lysine (K) in the linker. It is well known in the art that cholesteryl bromoacetate, as a modification moiety of the polypeptide, can also achieve modification to the polypeptide by performing a thioether-forming reaction with the side chain sulfhydryl group of the cysteine (C) in the linker.

X₁ is any one selected from the group consisting of acetyl (Ac), amino (NH₂), maleoyl, succinyl, tert-butoxycarbonyl, benzyloxy, another hydrophobic group and macromolecular carrier group. In some embodiments, X₁ is acetyl (Ac).

X₂ is any one selected from the group consisting of amino (NH₂), carboxyl, hydroxyl, amido, tert-butoxycarbonyl, another hydrophobic and macromolecular carrier group. In some embodiments, X₂ is amino (NH₂).

X₃ is any one selected from the group consisting of acetyl (Ac), amino (NH₂), maleoyl, succinyl, tert-butoxycarbonyl, benzyloxy, another hydrophobic group and macromolecular carrier group. In some embodiments, X₃ is acetyl (Ac).

X₄ is any one selected from the group consisting of amino (NH₂), carboxyl, hydroxyl, amido, tert-butoxycarbonyl, another hydrophobic group and macromolecular carrier group. In some embodiments, X₄ is amino (NH₂).

Abbreviations of amino acids in the polypeptide (polypeptide P1 or polypeptide P2) have the meanings well known in the art, and in addition to the amino acid abbreviations that have been previously described, the polypeptide of the present application further comprises: A (alanine), Q (glutamine), N (asparagine), etc. The amino acids are L-configured amino acids, and one or more (e.g., 2-5, 2-4, or 2-3) amino acid residues in the polypeptide may also be replaced by amino acids with chemically similar properties, such as L-configured amino acid(s), D-configured amino acid(s), artificially modified amino acid(s), or rare amino acid(s) present in nature, to improve the bioavailability, stability, and/or antiviral activity of the polypeptide, wherein the D-configured amino acid refers to an amino acid corresponding to a L-configured amino acid constituting a protein; the artificially modified amino acid refers to a common L-configured amino acid which constitutes a protein and is modified by means of methylation, phosphorylation or the like; and the rare amino acid present in nature includes an uncommon amino acids constituting a protein and an amino acid not constituting a protein, for example, 5-hydroxylysine, methylhistidine, gamma aminobutyric acid, and homoserine.

The amino acids in the polypeptide (polypeptide P1 or polypeptide P2) may undergo substitution, addition, or deletion of one or more other amino acids, and still has the activity of potently inhibiting HIV. The substitution of amino acids refers to the replacement of an amino acid residue at a position in the polypeptide sequence with another amino acid, preferably with a conservative amino acid; the addition of amino acids refers to the insertion of additional amino acid residue(s) at the N-terminus or C-terminus or other appropriate position of the polypeptide sequence, and the inserted amino acid residues may be fully or partially contiguous, or noncontiguous to each other; the deletion of amino acids refers to the removal of one or more amino acid residues in a polypeptide sequence, provided that the modified polypeptide has the activity for inhibiting HIV.

The so-called "conservative amino acid" or "conservation of amino acids" has the meanings well known in the art. For example, amino acids are classified into acidic amino acids, basic amino acids and neutral amino acids according to the numbers of amino groups and carboxyl groups contained in the amino acid molecules, wherein the acidic amino acids refer to E and D (aspartic acid), the basic amino acids refer to K, R (arginine) and H (histidine), and the neutral amino acids refer to A, L, I, V, C, Y (tyrosine), G (glycine), M (methionine), S (serine), T (threonine), F (phenylalanine), W (tryptophan) and P (proline). For another example, amino acids are classified into hydrophilic amino acids (D, E, H, K, Q, R, S, T) and hydrophobic amino acids (A, F, I, L, M, P, V, W, Y) according to the hydrophilicity and the hydrophobicity. For another example, hydrophilic uncharged amino acids refer to N, Q, S and T; aliphatic uncharged amino acids refer to A, L, I, V and G; nonpolar uncharged amino acids refer to C, M and P; aromatic amino acids refer to Y, F and W. For another example, amino acids containing alcohol group include S and T; aliphatic amino acids include L, I, V and M; cycloalkenyl-related amino acids include F, H, W and Y. These amino acids with similar sizes, shapes, charges, and chemical properties including the capability of forming covalent bond or hydrogen bond, are generally considered as conservative amino acids.

Specifically, the polypeptide P1 is the following (c1) or (c2) or (c3) or (c4) or (c5) or (c6):
(c1) a polypeptide with a sequence as set forth in SEQ ID NO: 1 in the sequence listing;
(c2) a polypeptide with a sequence as set forth in SEQ ID NO: 5 in the sequence listing;
(c3) a polypeptide with a sequence as set forth in SEQ ID NO: 6 in the sequence listing;
(c4) a polypeptide with a sequence as set forth in SEQ ID NO: 9 in the sequence listing;
(c5) a polypeptide with antiviral activity obtained by substitution and/or deletion and/or addition of amino acid residue on the basis of the polypeptide of any one of (c1) to (c4);
(c6) a polypeptide with a sequence identity of at least 60%, 65%, 70%, 75%, 80%, 85%, 90%, 93%, 94%, 95%, 96%, 97%, 98% or 99% as compared with the polypeptide of any one of (c1) to (c4) and having antiviral activity.

Specifically, the polypeptide P1 is the following (c1) or (c2) or (c3) or (c4) or (c5):
(c1) a polypeptide with the sequence as set forth in SEQ ID NO: 1 in the sequence listing;
(c2) a polypeptide with the sequence as set forth in SEQ ID NO: 5 in the sequence listing;
(c3) a polypeptide with the sequence as set forth in SEQ ID NO: 6 in the sequence listing;
(c4) a polypeptide with antiviral activity obtained by substitution and/or deletion and/or addition of amino acid residue on the basis of the polypeptide of any one of (c1) to (c3);
(c5) a polypeptide with a sequence identity of at least 60%, 65%, 70%, 75%, 80%, 85%, 90%, 93%, 94%, 95%, 96%, 97%, 98% or 99% as compared with the polypeptide of any one of (c1) to (c3) and having antiviral activity.

Specifically, the polypeptide P1 has the sequence as set forth in SEQ ID NO. 1, 5 or 6.

Specifically, the polypeptide P2 is the following (d1) or (d2) or (d3) or (d4):
(d1) a polypeptide with a sequence as set forth in SEQ ID NO: 7 in the sequence listing;
(d2) a polypeptide with a sequence as set forth in SEQ ID NO: 8 in the sequence listing;
(d3) a polypeptide with antiviral activity obtained by substitution and/or deletion and/or addition of amino acid residue on the basis of the polypeptide of any one of (d1) to (d2);
(d4) a polypeptide with a sequence identity of at least 60%, 65%, 70%, 75%, 80%, 85%, 90%, 93%, 94%, 95%, 96%, 97%, 98% or 99% as compared with the polypeptide of any one of (d1) to (d2) and having antiviral activity.

Specifically, the polypeptide P2 has the sequence as set forth in SEQ ID NO. 7 or 8.

The present inventor team has demonstrated with more than ten years of research experiences that polypeptides derived from substitutions, additions or deletions of amino acids in the parent polypeptide (polypeptide P1 or polypeptide P2) with a sequence identity of up to about 60%, 70%, 80%, 90% or 95% as inhibitors, may still have potent antiviral activity.

Accordingly, the present application also provides a polypeptide which is the following (a1) or (a2) or (a3):
(a1) a polypeptide with the amino acid sequence "EXEELEKKIEELLKKAEEQQKKNEEELKKLEK", wherein X is isoleucine, valine or leucine;
(a2) a polypeptide with antiviral activity obtained by substitution and/or deletion and/or addition of amino acid residue on the basis of (a1);
(a3) a polypeptide with a sequence identity of at least 60%, 65%, 70%, 75%, 80%, 85%, 90%, 93%, 94%, 95%, 96%, 97%, 98%, or 99% as compared with (a1) and having antiviral activity; or
the polypeptide is the following (b1) or (b2) or (b3):
(b1) a polypeptide with the amino acid sequence "EXEELEKKIEELLKKAEEQQKKNEEELKKLEKX", wherein "X" as the 2^{nd} amino acid residue is isoleucine, valine or leucine, and "X" as the 33^{rd} amino acid residue is lysine or cysteine;
(b2) a polypeptide with antiviral activity obtained by substitution and/or deletion and/or addition of amino acid residue on the basis of (b1);
(b3) a polypeptide with a sequence identity of 60%, 65%, 70%, 75%, 80%, 85%, 90%, 93%, 94%, 95%, 96%, 97%, 98%, or 99% as compared with (b1) and having antiviral activity.

In some embodiments, the polypeptide P1 is the following (c1) or (c2) or (c3) or (c4) or (c5) or (c6):
(c1) a polypeptide with the sequence as set forth in SEQ ID NO: 1 in the sequence listing;
(c2) a polypeptide with the sequence as set forth in SEQ ID NO: 5 in the sequence listing;
(c3) a polypeptide with the sequence as set forth in SEQ ID NO: 6 in the sequence listing;
(c4) a polypeptide with the sequence as set forth in SEQ ID NO: 9 in the sequence listing;
(c5) a polypeptide with antiviral activity obtained by substitution and/or deletion and/or addition of amino acid residue on the basis of the polypeptide of any one of (c1) to (c4);
(c6) a polypeptide with a sequence identity of at least 60%, 65%, 70%, 75%, 80%, 85%, 90%, 93%, 94%, 95%, 96%, 97%, 98% or 99% as compared with the polypeptide of any one of (c1) to (c4) and having antiviral activity.

In some embodiments, the polypeptide is the following (c1) or (c2) or (c3) or (c4) or (c5):
(c1) a polypeptide with the sequence as set forth in SEQ ID NO: 1 in the sequence listing;
(c2) a polypeptide with the sequence as set forth in SEQ ID NO: 5 in the sequence listing;
(c3) a polypeptide with the sequence as set forth in SEQ ID NO: 6 in the sequence listing;
(c4) a polypeptide with antiviral activity obtained by substitution and/or deletion and/or addition of amino acid residue on the basis of the polypeptide of any one of (c1) to (c3);
(c5) a polypeptide with a sequence identity of at least 60%, 65%, 70%, 75%, 80%, 85%, 90%, 93%, 94%, 95%, 96%, 97%, 98% or 99% as compared with the polypeptide of any one of (c1) to (c3) and having antiviral activity; or
the polypeptide is the following (d1) or (d2) or (d3) or (d4):
(d1) a polypeptide with the sequence as set forth in SEQ ID NO: 7 in the sequence listing;
(d2) a polypeptide with the sequence as set forth in SEQ ID NO: 8 in the sequence listing;
(d3) a polypeptide with antiviral activity obtained by substitution and/or deletion and/or addition of amino acid residue on the basis of the polypeptide of any one of (d1) to (d2);
(d4) a polypeptide with a sequence identity of at least 60%, 65%, 70%, 75%, 80%, 85%, 90%, 93%, 94%, 95%, 96%, 97%, 98% or 99% as compared with the polypeptide of any one of (d1) to (d2) and having antiviral activity.

In some embodiments, the substitution of amino acid residue as described in the present application is a conservative substitution.

In the present application, the term "conservative substitution" refers to amino acid substitutions which would not disadvantageously affect or change the essential properties of a protein/polypeptide comprising the amino acid sequence. For example, a conservative substitution may be introduced by standard techniques known in the art such as site-directed mutagenesis and PCR-mediated mutagenesis. Conservative substitutions of amino acid include substitutions wherein an amino acid residue is substituted with another amino acid residue having a similar side chain, for example, a residue physically or functionally similar (such as, having similar size, shape, charge, chemical property including the capability of forming covalent bond or hydrogen bond, etc.) to the corresponding amino acid residue. The families of amino acid residues having similar side chains have been defined in the art. These families include amino acids having basic side chains (for example, lysine, arginine and histidine), amino acids having acidic side chains (for example, aspartic acid and glutamic acid), amino acids having uncharged polar side chains (for example, glycine, asparagine, glutamine, serine, threonine, tyrosine, cysteine, and tryptophan), amino acids having nonpolar side chains (for example, alanine, valine, leucine, isoleucine, proline, phenylalanine, and methionine), amino acids having β-branched side chains (such as threonine, valine, and isoleucine) and amino acids having aromatic side chains (for example, tyrosine, phenylalanine, tryptophan, and histidine). Therefore, generally a conservative substitution refers to a substitution of a corresponding amino acid residue with another amino acid residue from the same side-chain family. Methods for identifying the conservative substitutions of amino acids are well known in the art.

The lipopeptides as described in the present application may contain one or more chiral centers, and/or double bonds and structures like that, and thus may exist as stereoisomers, including isomers of double bonds (e.g., geometrical isomers), enantiomers (opticalisomeride), or diastereomers. Accordingly, any chemical structures within the scope as described here, whether partially or wholly containing the above similar structures, includes all possible enantiomers and diastereomers of the lipopeptides, including any one pure stereoisomer (e.g., pure geometrical isomers, pure enantiomers, or pure diastereomers), and any one mixture of these stereoisomers. One skilled in the art, by utilizing separation techniques or asymmetric synthesis methods, can also further resolve mixtures of these racemates and stereoisomers into the enantiomers or stereoisomers of the constituents thereof. The lipopeptides include, but are not limited to, various optical isomers, racemates and/or other mixtures. In the above case, a single enantiomer or diastereomer, e.g., an optically active isomer, can be obtained by an asymmetric synthesis method or a racemate resolution method. The resolution of racemates can be accomplished in a variety of ways, e.g., routine recrystallization with resolution-assisting reagents, or chromatography. In addition, the lipopeptides also include cis-and/or trans-isomers with double bonds.

The lipopeptides of the present application include, but are not limited to, all of the various pharmaceutically acceptable forms of the lipopeptides. These pharmaceutically acceptable forms include various pharmaceutically acceptable salts, solvates, hydrates, complexes, chelates, non-covalent complexes, prodrugs based on the above substances, and any mixtures of the above forms.

The pharmaceutically acceptable salts of the present application include acetate, lactobionate, benzenesulfonate, laurate, benzoate, malate, bicarbonate, maleate, bisulfate, mandelate, bitartrate, mesylate, borate, methylbromide, bromide, methylnitrate, calcium edetate, methylsulfate, camsylate, mucate, carbonate, napsylate, chloride, nitrate, clavulanate, N-methylglucamine, citrate, ammonium salt, dihydrochloride, oleate, edetate, oxalate, edisylate, pamoate, embonate, estolate, palmitate, esylate, pantothenate, fumarate, phosphate/diphosphate, gluceptate, polygalacturonate, gluconate, salicylate, glutamate, stearate, glycollylarsanilate, sulfate, hexylresorcinate, subacetate, hydrabamine, succinate, hydrobromide, tannate, hydrochloride, tartrate, hydroxynaphthoate, teoclate, iodide, tosylate, triethiodide, lactate, and valerate, etc. Depending on the use, the pharmaceutically acceptable salt may be formed from cations such as sodium, potassium, and bismuth, or may be formed from a base such as ammonia, ethylenediamine, N-methyl-glutamine, lysine, arginine, ornithine, choline, N,N'-dibenzylethylenediamine, chloroprocaine, diethanolamine, procaine, diethylamine, piperazine, tris (hydroxymethyl) aminomethane and tetramethylammonium hydroxide. These salts may be prepared by standard methods, for example, by a reaction of a free acid with an organic or inorganic base. In the presence of a basic group (e.g., an amino group), an acid salt such as a hydrochloride, a hydrobromide, an acetate, and a pamoate may be used as a form of a drug; in the presence of an acidic group or an alcohol group, a pharmaceutically acceptable ester such as an acetate, a maleate, and a pivaloyloxymethyl, and an ester known in the literatures for improving solubility and hydrolyzability may be used as a form of sustained release drug or prodrug.

The present application also provides a conjugate comprising the polypeptide of the present application and a modification moiety. In some embodiments, the modification moiety is linked to the N-terminus or C-terminus of the polypeptide, optionally through a linker. In some embodiments, the modification moiety is a terminal protecting group. The terminal protecting group of the polypeptide comprises an N-terminal protecting group and/or a C-terminal protecting group. As is well known, the N-terminal protecting group may be any one selected from the group consisting of acetyl (Ac), amino (NH₂), maleoyl, succinyl, tert-butoxycarbonyl, benzyloxy, another hydrophobic group and macromolecular carrier group. As well known, the C-terminal protecting group may be any one selected from the group consisting of amino (NH₂), carboxyl, hydroxyl, amido, tert-butoxycarbonyl, another hydrophobic and macromolecular carrier group.

The present application also provides an isolated nucleic acid, which encodes the polypeptide of the present application.

The present application also provides a vector comprising the isolated nucleic acid of the present application. Vectors useful for inserting a polynucleotide of interest are well known in the art and include, but are not limited to, cloning vectors and expression vectors. In one embodiment, the vector is, for example, plasmid, cosmid, phage.

The present application also provides a host cell comprising the isolated nucleic acid and/or the vector of the present application. Such host cells include, but are not limited to, prokaryotic cells such as E. coli cells, and eukaryotic cells such as yeast cells, insect cells, plant cells, and animal cells (e.g., mammalian cells such as mouse cells and human cells). The host cell of the present application may also be a cell line.

The present application also provides a multimer, which is the following (e1) or (e2) or (e3) or (e4) or (e5) or (e6):
(e1) a multimer formed from any one of the lipopeptides;
(e2) a multimer formed from any one of the pharmaceutically acceptable salts;
(e3) a multimer formed from any one of the derivatives;
(e4) a multimer formed from any one of the hydrates;
(e5) a multimer formed from any one of the solvates;
(e6) a multimer formed from any one of the polypeptides.

In some embodiments, the multimer is the following (e1) or (e2) or (e3):
(e1) a multimer formed from any one of the lipopeptides;
(e2) a multimer formed from any one of the pharmaceutically acceptable salts;
(e3) a multimer formed from any one of the derivatives.

The present application also provides a composition, comprising the lipopeptide, a pharmaceutically acceptable salt thereof, a solvate thereof, a hydrate thereof or a derivative thereof, or the polypeptide, or the conjugate, or the isolated nucleic acid, or the vector, or the host cell, or the multimer of the present application.

The present application also provides a pharmaceutical composition, comprising the lipopeptide, a pharmaceutically acceptable salt thereof, a solvate thereof, a hydrate thereof or a derivative thereof, or the polypeptide of the present application, optionally further comprising a pharmaceutically acceptable carrier. In some embodiments, the lipopeptide, a pharmaceutically acceptable salt thereof, a solvate thereof, a hydrate thereof or a derivative thereof or the polypeptide is present in an amount effective for treating a viral infection or a disease caused by viral infection. In some embodiments, the viral infection is an infection caused by a virus selected from the group consisting of HIV, SIV, and resistant strain thereof. In some embodiments, the HIV is HIV-1 and/or HIV-2. In some embodiments, the HIV is a T20 resistant virus. In some embodiments, the HIV is an LP-98 resistant virus. In some embodiments, the HIV is an LP-40 resistant virus. In some embodiments, the HIV is an LP-52 resistant virus. In some embodiments, the HIV is an SC29EK resistant virus. In some embodiments, the HIV is an SC22EK resistant virus. In some embodiments, the HIV is an MTSC22 resistant virus. In some embodiments, the HIV is an SFT resistant virus. In some embodiments, the viral infection is an infection caused by a virus selected from the group consisting of a T20 resistant virus, an LP-40 resistant virus, an LP-52 resistant virus, an SC29EK resistant virus, an SC22EK resistant virus, an MTSC22 resistant virus, and an SFT resistant virus. In some embodiments, the disease caused by viral infection is AIDS.

The present application also provides use of any one of the above lipopeptides or any one of the above pharmaceutically acceptable salts or any one of the above derivatives or the multimer, which is the following (f1) or (f2) or (f3) or (f4):
(f1) use in the manufacture of a viral membrane fusion inhibitor;
(f2) use in the manufacture of a medicament for preventing and/or treating a disease caused by viral infection;
(f3) use as a viral membrane fusion inhibitor;
(f4) use in prevention and/or treatment of a disease caused by viral infection.

The present application also provides use of any one of the above polypeptides, which is the following (f1) or (f2) or (f3) or (f4):
(f1) use in the manufacture of a viral membrane fusion inhibitor;
(f2) use in the manufacture of a medicament for preventing and/or treating a disease caused by viral infection;
(f3) use as a viral membrane fusion inhibitor;
(f4) use in prevention and/or treatment of a disease caused by viral infection.

The present application also provides a product comprising any one of the above lipopeptides or any one of the above pharmaceutically acceptable salts or any one of the above derivatives or the multimer, the product has the following function (g1) or (g2):
(g1) function as a viral membrane fusion inhibitor;
(g2) function of preventing and/or treating a disease caused by viral infection.

The present application also provides a product comprising any one of the above polypeptides, the product has the following function (g1) or (g2):
(g1) function as a viral membrane fusion inhibitor;
(g2) function of preventing and/or treating a disease caused by viral infection.

The product may also include a carrier.

The present application also provides use of the product, which is the following (g1) or (g2):
(g1) use as a viral membrane fusion inhibitor;
(g2) use for preventing and/or treating a disease caused by viral infection.

The application also provides the product as a viral membrane fusion inhibitor; or for preventing and/or treating a disease caused by viral infection.

The present application also provides a method for treating or/and preventing a viral infection in an animal, comprising administering any one of the above lipopeptides or any one of the above pharmaceutically acceptable salts or any one of the above derivatives or the multimer to the subject animal to inhibit viral infection in the animal.

The present application also provides a method for treating or/and preventing a disease caused by viral infection, comprising administering an effective amount of any one of the above lipopeptides or pharmaceutically acceptable salts thereof or solvates thereof or hydrates thereof or derivatives thereof or the polypeptide or the multimer or the pharmaceutical composition to a subject in need thereof.

Any one of the above viruses is HIV.

The virus is Human Immunodeficiency Virus (HIV) and/or Simian Immunodeficiency Virus (SIV).

In some embodiments, the virus is selected from the group consisting of HIV, SIV, and drug-resistant strain thereof.

Specifically, the HIV is HIV-1 and/or HIV-2.

Specifically, the HIV is a T20 resistant virus.

Specifically, the HIV is an LP-98 resistant virus.

Specifically, the virus is a T20 resistant virus and/or an LP-40 resistant virus and/or an LP-52 resistant virus and/or an SC29EK resistant virus and/or an SC22EK resistant virus and/or an MTSC22 resistant virus and/or an SFT resistant virus.

Specifically, any one of the above products may be a medicament or a vaccine.

In some embodiments, the HIV is a T20 resistant virus. In some embodiments, the HIV is an LP-40 resistant virus. In some embodiments, the HIV is an LP-52 resistant virus. In some embodiments, the HIV is an SC29EK resistant virus. In some embodiments, the HIV is an SC22EK resistant virus. In some embodiments, the HIV is an MTSC22 resistant virus. In some embodiments, the HIV is an SFT resistant virus.

In some embodiments, the animal is a mammal, e.g., a human.

In some embodiments, the viral infection is an infection caused by a virus selected from the group consisting of HIV, SIV, and resistant strain thereof.

In some embodiments, the viral infection is an infection caused by a virus selected from the group consisting of a T20 resistant virus, an LP-40 resistant virus, an LP-52 resistant virus, an SC29EK resistant virus, an SC22EK resistant virus, an MTSC22 resistant virus, and an SFT resistant virus.

In some embodiments, the disease caused by viral infection is AIDS.

In some embodiments, the subject is a mammal, e.g., a human.

In practice, the lipopeptide of the present application may be administered to a patient as a medicament either directly or in admixture with a suitable carrier or excipient for the purpose of treating and/or preventing HIV infection.

The material of the carrier is preferably a pharmaceutically acceptable carrier material, which includes, but is not limited to, water-soluble carrier material (e.g., polyethylene glycol, polyvinylpyrrolidone, organic acid, etc.), poorly soluble carrier material (e.g., ethyl cellulose, cholesterol stearate, etc.), enteric soluble carrier material (e.g., cellulose acetate phthalate, carboxymethyl cellulose, etc.), wherein the water-soluble carrier material is preferred. By using these materials, various preparation forms can be prepared, including but not limited to tablet, capsule, dripping pill, aerosol, pill, powder, solution, suspension, emulsion, granule, liposome, transdermal agent, buccal tablet, suppository, freeze-dried powder for injection, etc. The preparation form may be a conventional preparation, a sustained release preparation, a controlled release preparation and various microparticle delivery systems. In order to formulate a unit preparation form into a tablet, a wide variety of carriers known in the art may be used. Examples of carriers are, for example, diluent and absorbent, such as starch, dextrin, calcium sulfate, lactose, mannitol, sucrose, sodium chloride, glucose, urea, calcium carbonate, kaolin, microcrystalline cellulose, and aluminum silicate; wetting agent and binder, such as water, glycerol, polyethylene glycol, ethanol, propanol, starch slurry, dextrin, syrup, honey, glucose solution, gum arabic, gelatin paste, sodium carboxymethylcellulose, shellac, methylcellulose, potassium phosphate, and polyvinyl pyrrolidone; disintegrant, such as dried starch, alginate, agar powder, brown algae starch, sodium bicarbonate and citric acid, calcium carbonate, polyoxyethylene, sorbitol fatty acid ester, sodium dodecylsulfate, methyl cellulose, and ethyl cellulose; disintegration inhibitor, such as sucrose, glyceryl tristearate, cocoa butter, and hydrogenated oil; absorption promoters, such as quaternary ammonium salts, and sodium lauryl sulfate; lubricant, such as talc, silica, corn starch, stearate, boric acid, liquid paraffin, and polyethylene glycol. The tablet may also be further formulated into a coated tablet, such as sugar-coated tablet, film-coated tablet, enteric-coated tablet, or double or multiple layer tablet. In order to formulate a unit preparation form into a pill, a wide variety of carriers known in the art may be used. Examples of the carrier are, for example, diluent and absorbent, such as glucose, lactose, starch, cocoa butter, hydrogenated vegetable oil, polyvinyl pyrrolidone, Gelucire, kaolin, and talc; binder, such as gum arabic, tragacanth, gelatin, ethanol, honey, liquid sugar, rice paste, and flour paste; disintegrant, such as agar powder, dried starch, alginate, sodium dodecyl sulfate, methyl cellulose, and ethyl cellulose. In order to formulate a unit preparation form into a suppository, a wide variety of carriers known in the art may be used. Examples of the carrier are, for example, polyethylene glycol, lecithin, cocoa butter, higher alcohol, higher alcohol ester, gelatin, and semi-synthetic glyceride. In order to formulate a unit preparation form into a preparation for injection such as a solution, an emulsion, a freeze-dried powder and a suspension, all conventional diluents may be used, for example, water, ethanol, polyethylene glycol, 1,3-propanediol, ethoxylated isostearyl alcohol, polyoxylated isostearyl alcohol, and polyoxyethylene sorbitan fatty acid ester. In addition, in order to prepare an isotonic injection, to a preparation for injection, sodium chloride, glucose, or glycerin in a suitable amount may be added, and furthermore, conventional cosolvent, buffer, pH-adjusting agent may also be added. Besides, if required, coloring agent, preservative, perfume, flavor, sweeting agent, and other material may optionally be added to the pharmaceutical preparation. The above preparation forms may be administered by injection including subcutaneous injection, intravenous injection, intramuscular injection, intracavitary injection and the like, intraluminal administration such as transrectal and vaginal administration, respiratory administration such as nasal administration; and mucosal administration. Among the above administration routes, the administration by injection is preferred.

The administration dose of the lipopeptide or polypeptide of the present application depends on various factors, for example, the nature and severity of a disease to be prevented or treated, the gender, age, weight and individual response of a patient or animal, a specific ingredient used, an administration route, and an administration frequency etc. The above dose may be administered in a single-unit dosage form or multiple- (e.g., two, three or four) unit dosage forms.

The lipopeptide or polypeptide of the present application may be directly used alone for the treatment or prevention of HIV infected patient, or may be used in combination with one or more antiviral drugs, to achieve the purpose of improving overall therapeutic effects. The antiviral drugs include, but are not limited to, reverse transcriptase inhibitors, protease inhibitors, entry inhibitors, integration inhibitors, maturation inhibitors, and the like. The above reverse transcriptase inhibitors may be one or more inhibitors selected from the group consisting of AZT, 3TC, ddI, d4T, ddT, TDF, Abacavir, Nevirapine, Efavirenz, Delavirdine, Azvudine, Ainuovirine and the like; the above protease inhibitors may be one or more inhibitors selected from the group consisting of Saquinavir mesylate, Indinavir, Ritonavir, Amprenavir, Nelfinavir mesylate and the like; the above invasion inhibitors may be one or more inhibitors selected from the group consisting of Maraviroc, TAK-779, T20, T2635, Sifuvirtide, Albuvirtide, and the like; the above integration inhibitors may be one or more inhibitors selected from the group consisting of Raltegravir, Dolutegravir, Elvitegravi and the like.

A specific therapeutically effective dose level for any individual patient will depend on various factors, including disorder being treated and the severity thereof; the activity of specific active ingredient used; a specific composition used; the age, weight, general health, gender and diet of a patient; an administration time, an administration route and an excretion rate of a specific active ingredient used; a duration of treatment; a drug used together with the specific active ingredient used in combination or simultaneously; and similar factors well known in the medical field. For example, it is common practice in the art to start with a dosage of an active ingredient at a level below that required to achieve a desired therapeutic effect, and to gradually increase the dosage until the desired effect is achieved. In general, the lipopeptide or polypeptide of the present application may be administered to a mammal, particularly a human, in a dosage of between 0.001 mg/kg body weight/day and 1000 mg/kg body weight/day, such as between 0.01 mg/kg body weight/day and 100 mg/kg body weight/day, further such as between 0.1 mg/kg body weight/day and 10 mg/kg body weight/day.

The lipopeptide of the present application has a potent inhibitory activity against HIV-1, HIV-2 and SIV, and especially, its inhibitory activity against T20 resistant and LP-98 resistant stains is significantly increased. Hence, the lipopeptide of the present application is a broad-spectrum inhibitor and it can be used for treating and preventing diseases caused by HIV-1, HIV-2 and SIV infections. The present application has great application value for the preventions and treatments of AIDS.

The existing research suggests that the hydrophobic pocket of NHR helix in HIV fusion protein gp41 is an important drug target, while the PBD sequence (W₁MEW₂DREI) of the eight amino acids at the N-terminus of the CHR helix plays an important role in the design of a polypeptide HIV membrane fusion inhibitor. In particular, the interaction of two highly conservative tryptophans (designated as W₁ and W₂) and one isoleucine (I) with the hydrophobic pocket is considered as the key for the inhibitor to exert its antiviral activity by binding the NHR target. In view of the importance of the PBD sequence, the present application is devoted to the design and identification of potent HIV membrane fusion inhibitors containing the PBD amino acid. The inventors surprisingly find through systematic studies that lipopeptides only containing two terminal amino acids EI of the PBD sequence have significantly enhanced antiviral activity, while by further adding corresponding amino acid motifs containing W₂ or W₁, the antiviral activity of the lipopeptides is gradually reduced. This important discovery provides innovative ideas and design strategies for the researches and developments of novel HIV membrane fusion inhibitor medicaments.

### Brief Description of the Drawings

FIG. 1 is a diagram showing the sequence comparisons of T20, C34, SFT, ABT and LP-98 in the Background Art.
FIG. 2 is a diagram of the results of Example 2 (sequence structure of the lipopeptide and the identifications to its activity against HIV-1).
FIG. 3 is a diagram of the results of Example 3 (inhibitory effects of the representative lipopeptides against various HIV-1 resistant strains, wherein the bold numbers in the parentheses indicating the fold number by which the activities of the lipopeptides are increased as compared to LP-98).
FIG. 4 is a diagram of the results of Example 4 (inhibitory effects of the representative lipopeptides against HIV-2 and SIV).
FIG. 5 is a diagram of the results of Example 5 (helical structural features of the representative lipopeptides and interactions thereof with the target sequence; A: α-helix contents of the lipopeptide inhibitors alone; B: helical stabilities of the lipopeptide inhibitors alone; C: α-helix contents of the complexes of lipopeptide inhibitors and N42 polypeptide; D: helical stabilities of the complexes of lipopeptide inhibitors and N42 polypeptide).

### Sequence Information

Information on the sequences involved in the present application is provided in the following Table 1.

**Table 1: Descriptions of sequences**

| SEQ ID NO. | Names/descriptions | Sequence information |
|---|---|---|
| 1 | Polypeptide sequence in LP-101 | E**I**EELEKKIEELLKKAEEQQKKNEEELKKLEK |
| 2 | Polypeptide sequence in LP-102 | WEQK**I**FFTFKKTFFTTKKAEEQQKKNEEELKKLEK |
| 3 | Polypeptide sequence in LP-103 | WDRE**I**EELEKKIEELLKKAEEQQKKNEEELKKLEK |
| 4 | Polypeptide sequence in LP-104 | WEEWEKK**I**EELEKKIEELLKKAEEQQKKNEEELKKLEK |
| 5 | Polypeptide sequence in LP-105 | E**V**EELEKK**I**EELLKKAEEQQKKNEEELKKLEK |
| 6 | Polypeptide sequence in LP-106 | E**L**EELEKKIEELLKKAEEQQKKNEEELKKLEK |
| 7 | Polypeptide sequence in LP-107 | E**I**EELEKKIEELLKKAEEQQKKNEEELKKLEKK |
| 8 | Polypeptide sequence in LP-108 | F**I**FFTFKKTFFTTKKAEEQQKKNEEELKKLEKC |
| 9 | Polypeptide sequence in LP-109 | E**I**EELEKKIEELLKKAEEQQKKNEEELKKLEK |
| 10 | Polypeptide sequence in LP-98 | YEQKIEELLKKAEEQQKKNEEELKKLEK |
| 11 | Polypeptide N42 | STMGAASMTLTVQARQLLSGIVQQQNNLLRAIEAQQHLLQLT |

### Detailed Description of the Preferred Embodiment

The present application will be described in detail below with reference to the specific embodiments, and the examples provided here are only for illustrating the present application but not for limiting the scope of the present application. The examples provided below serve as a guidance for one of ordinary skill in the art to make modifications, but not construct any limits on the present application in any way. One skilled in the art can refer to the present disclosures to appropriately modify related parameters. Specifically, it is necessary to indicate that all similar substitutions or changes are obvious for one skilled in the art, and all of them are considered to be within the scope of the present application. The method of the present application has been described with preferred examples, and it will be apparent that related person can achieve and apply the techniques of the present application by making modifications or appropriate changes or combinations to the compounds and preparation methods as described herein, without departing from the contents, spirits and scope of the present application.

The experimental methods in the following examples, unless otherwise illustrated, each are conventional methods, and they are carried out according to the techniques or conditions described in literature in this field or according to the product instructions. The materials, reagents and the like used in the following examples, unless otherwise illustrated, each are commercially available. Unless otherwise illustrated, the quantitative tests in the following examples are carried out in triplicate, and the results are averaged.

### Example 1. Preparation of Lipopeptides

The following four lipopeptides were prepared respectively:
lipopeptide LP-101 containing two terminal amino acids E and I of PBD (containing one amino acid inserted into the NHR pocket);
lipopeptide LP-102 containing five amino acids W₂EQKI (containing two amino acids inserted into the NHR pocket);
lipopeptide LP-103 containing five amino acids W₂DREI (containing two amino acids inserted into the NHR pocket);
lipopeptide LP-104 containing all the eight PBD amino acids W₁EEW₂EKKI (containing three amino acids inserted into the NHR pocket).
W₁ referred to the first W from the N terminus of the PBD, and W₂ referred to the second W from the N terminus of the PBD.

The antiviral activities of the above four lipopeptides were identified and other five lipopeptides were obtained by making further modifications.

The following five lipopeptides were prepared respectively:
lipopeptide LP-105 (as compared to lipopeptide LP-101, the amino acid I inserted into the NHR pocket was replaced with amino acid V);
lipopeptide LP-106 (as compared to lipopeptide LP-101, the amino acid I inserted into the NHR pocket was replaced with amino acid L);
lipopeptide LP-107 (as compared to lipopeptide LP-101, an amino acid K was added to the C-terminus of the peptide chain);
lipopeptide LP-108 (as compared to lipopeptide LP-101, an amino acid C was added to the C-terminus of the peptide chain);
lipopeptide LP-109 (as compared to lipopeptide LP-101, the modification group at the C-terminus of the peptide chain was replaced with stearic acid).

Lipopeptide LP-98 was prepared.

The above nine prepared lipopeptides LP-98 to LP-108 each had a cholesterol modification, and the LP-109 had a stearic acid modification. The cholesterol modifications of lipopeptide LP-98, lipopeptide LP-101, lipopeptide LP-102, lipopeptide LP-103, lipopeptide LP-104, lipopeptide LP-105, lipopeptide LP-106 and lipopeptide LP-107 each were achieved by the amidation reaction of the side chain amino group of the C-terminal lysine of the peptide chain. The cholesterol modification of the lipopeptide LP-108 was achieved by a highly chemically selective thioether-forming reaction between the side chain sulfhydryl group of the C terminal cysteine of the peptide chain and a cholesteryl bromoacetate. The amino terminal of all ten lipopeptides linked an acetyl (Ac) as an amino-terminal protecting group, and the carboxyl terminal linked an amino (NH₂) as a carboxy-terminal protecting group. The stearic acid modification of the lipopeptide LP-109 was achieved by the amidation reaction of the side chain amino group of the C-terminal lysine of the peptide chain.

The structures of the ten lipopeptides were shown as follows:
LP-98: Ac-YEQKIEELLKKAEEQQKKNEEELKKLEK(chol)-NH₂;
LP-101: Ac-EIEELEKKIEELLKKAEEQQKKNEEELKKLEK(chol)-NH₂;
LP-102: Ac-WEQKIEELEKKIEELLKKAEEQQKKNEEELKKLEK(chol)-NH₂;
LP-103: Ac-WDREIEELEKKIEELLKKAEEQQKKNEEELKKLEK(chol)-NH₂;
LP-104: Ac-WEEWEKKIEELEKKIEELLKKAEEQQKKNEEELKKLEK(chol)-NH₂;
LP-105: Ac-EVEELEKKIEELLKKAEEQQKKNEEELKKLEK(chol)-NH₂;
LP-106: Ac-ELEELEKKIEELLKKAEEQQKKNEEELKKLEK(chol)-NH₂;
LP-107: Ac-EIEELEKKIEELLKKAEEQQKKNEEELKKLEKK(chol)-NH₂;
LP-108: Ac-EIEELEKKIEELLKKAEEQQKKNEEELKKLEKC(chol)-NH₂;
LP-109: Ac-EIEELEKKIEELLKKAEEQQKKNEEELKKLEKC(C18)-NH₂.
(chol) referred to that the C-terminal amino acid was modified with cholesterol, for example, cholesteryl hemisuccinate or cholesteryl bromoacetate.
(C18) referred to that the C-terminal amino group was modified with stearic acid.

The amino acid sequences of the polypeptide peptide chains of the ten lipopeptides were shown as follows (from N-terminus to C-terminus):
LP-98 (SEQ ID NO: 10): YEQKIEELLKKAEEQQKKNEEELKKLEK;
LP-101 (SEQ ID NO: 1): EIEELEKKIEELLKKAEEQQKKNEEELKKLEK;
LP-102 (SEQ ID NO: 2): WEQKIEELEKKIEELLKKAEEQQKKNEEELKKLEK;
LP-103 (SEQ ID NO: 3): WDREIEELEKKIEELLKKAEEQQKKNEEELKKLEK;
LP-104 (SEQ ID NO: 4): WEEWEKKIEELEKKIEELLKKAEEQQKKNEEELKKLEK;
LP-105 (SEQ ID NO: 5): EVEELEKKIEELLKKAEEQQKKNEEELKKLEK;
LP-106 (SEQ ID NO: 6): ELEELEKKIEELLKKAEEQQKKNEEELKKLEK;
LP-107 (SEQ ID NO: 7): EIEELEKKIEELLKKAEEQQKKNEEELKKLEKK;
LP-108 (SEQ ID NO: 8): EIEELEKKIEELLKKAEEQQKKNEEELKKLEKC;
LP-109 (SEQ ID NO: 9): EIEELEKKIEELLKKAEEQQKKNEEELKKLEKC.

The lipopeptides may be prepared by any conventional method known in the prior art. Illustratively, the following methods may be used to prepare the lipopeptides:

### I. Chemical Reagents Required in Preparation Process

All chemical reagents, such as various Fmoc amino acids, N,N'-diisopropylcarbodiimide (DIC), 1-hydroxybenzotriazole (HOBt), N,N-dimethylformamide (DMF), hexahydropyridine (PIPE), ninhydrin, acetic anhydride (Ac₂O), N,N-diisopropylethylamine (DIEA), hydrazine hydrate, cholesteryl hemisuccinate, trifluoroacetic acid (TFA), ethanedithiol (EDT), thioanisole (TA), triisopropylsilane (TIPS), phenol and the like, each are purchased from major chemical reagent suppliers and are not further purified prior to use. Amino acid-protecting raw materials used in the synthesis of polypeptides include Fmoc-Lys(Dde)-OH, Fmoc-Lys(Boc)-OH, Fmoc-Glu(OtBu)-OH, Fmoc-Ile-OH, Fmoc-Leu-OH, Fmoc-Ala-OH, Fmoc-Gln(Trt)-OH, Fmoc-Asn(Trt)-OH, Fmoc-Val-OH, Fmoc-Cys(Trt)-OH, and Fmoc-Tyr(tBu)-OH. The abbreviations among them have well known meanings, for example: Fmoc is 9-fluorenylmethoxycarbonyl, Dde is 1-(4,4-dimethyl-2,6-dioxocyclohexylene) ethyl, Boc is t-butoxycarbonylacyl, tBu is t-butyl, OtBu is t-butoxy, Trt is trityl, and Pbf is (2,3-dihydro-2,2,4,6,7-pentamethylbenzofuran-5-yl)sulfonyl.

### II. Synthesis of Peptide Resins

Rink Amide MBHA resin was used as a carrier resin, and it was sequentially coupled with corresponding protected amino acids in the amino acid sequences of polypeptides through Fmoc deprotection and a coupling reaction, to prepare peptide resins.

### 1. Coupling of the First Protected Amino Acid to Main Chain

0.3 mmol of the first protected amino acid Fmoc-Lys(Dde)-OH or Fmoc-Cys(Trt)-OH and 0.3 mmol of HOBt were taken and dissolved with an appropriate amount of DMF; 0.3 mmol of DIC was additionally taken, slowly added into the DMF solution of the protected amino acid under shaking, and the resulting mixture was reacted at room temperature for 5 minutes under shaking, to obtain an activated protected amino acid solution, which was set aside for later use.

0.1 mmol of Rink Amide MBHA resin (0.35 mmol/g×0.3 g) were taken and deprotected with a 25% PIPE/DMF solution (volume ratio) for 20 min (twice), after washing and filtering, to obtain an Fmoc-removed resin.

The activated first protected amino acid solution was added into the Fmoc-removed resin to carry out a coupling reaction for 60 minutes, after filtering and washing, to obtain a resin containing the first protected amino acid Fmoc-Lys(Dde) or Fmoc-Cys(Trt) -OH.

### 2. Coupling of Other Protected Amino Acids to Main Chain

By using the same method as above for coupling the first protected amino acid into the main chain, the corresponding other protected amino acids were sequentially coupled to the polypeptide, to obtain resins containing main chain amino acids. At last, the N-terminus was capped by acetylation with 0.3 mmol of Ac₂O + 0.6 mmol of DIEA, to complete the synthesis of the main chain. After the reaction in each of the above steps, the reaction was controlled by Kaiser Test, and if the condensation reaction of certain amino acid was incomplete, the condensation was repeated once until the desired target peptide segment was obtained.

### 3. Coupling of Side Chains

(1) The resin was treated with 2% hydrazine hydrate/DMF solution (volume ratio) in a volume as small as possible to remove the side chain Dde protecting group of the C-terminal lysine (for 10 minutes, twice), after filtering and washing, to obtain a Dde-removed resin, which was set aside for later use.
(2) Cholesterol Modification of Polypeptides

The method for coupling cholesterol to the lysine side chain was described in the literature (Xue et al, 2022) published by the inventors. 0.3 mmol of cholesteryl hemisuccinate and 0.3 mmol of HOBt were taken and dissolved with an appropriate amount of DMF; 0.3 mmol of DIC was additionally taken, slowly added into the DMF solution of the cholesteryl hemisuccinate and HOBt, and the resulting mixture was reacted at room temperature for 5 minutes under shaking. The prepared solution comprising cholesteryl hemisuccinate, HOBt and DIC was added to the obtained Dde-removed resin to carry out a coupling reaction for 60 minutes, after filtering, washing and drying, to obtain a peptide resin.

The method for coupling cholesterol to the cysteine side chain was described in the literature (Dwyer et al, 2007) published by Dwyer et al. The method had been routinely used by the present inventors in the laboratory to prepare cholesterol-modified lipopeptides such as C34-Chol, LP-83, LP-86, and LP-97 (Xue et al, 2022; Zhu et al, 2019). First, cholesteryl bromoacetate was synthesized according to the technical route as described in the literature, and then it was grafted to a polypeptide chain by means of a highly chemically selective thioether-forming reaction which is carried out between the side chain thiol group of the C-terminal cysteine of the polypeptide and the cholesteryl bromoacetate, that was, after a crude polypeptide product was synthesized in a conventional manner, it was dissolved in pure DMSO, 1 equivalent of cholesterol bromoacetate dissolved in a small amount of trifluoroacetic acid (TFA) was added thereto, and then pure diisopropylethylamine (DIEA) was added to adjust pH to alkaline. The reaction was monitored by RP-HPLC and was generally completed in 1 hour.

### (3) Stearoylation of Polypeptides

0.3 mmol of stearoyl chloride and 0.6 mmol of DIEA were taken and dissolved in an appropriate amount of DMF, slowly added into the Dde-removed obtained in the step (1), and the resulting mixture was reacted at room temperatures for 60 minutes under shaking, after filtering, washing and drying, to obtain a peptide resin.

### III. Preparation of Crude Products

The above peptide resin was taken and added to a cleavage reagent (the cleavage agent 15 mL/g resin), and mixed well, the resulting mixture was reacted at 30 °C for 3 hours under shaking, thereby cleaving the polypeptide of interest from the resin and removing the side chain protecting group. The filtrate of the reaction mixture was collected. The resin was further washed with a small quantity of TFA/DCM for three times, and after the filtrates was combined, the combined filtrates was precipitated by adding anhydrous ether and centrifuged. The filter cake was further washed and precipitated with cold anhydrous ether for second times, then dried under vacuum, to obtain white powder, i.e., the lipopeptide crude product; the cleavage reagent had the following composition: trifluoroacetic acid : 1,2-ethanedithiol : thioanisole : phenol : H₂O : triisopropylsilane = 68.5 : 10 : 10 : 5 : 3.5 : 1 (v/v).

### IV. Preparation of Pure Products

The above lipopeptide crude product was taken and dissolved by adding water/acetonitrile, and the resulting mixture was centrifuged to remove insoluble substances, and was set aside for later use. A reversed-phase high performance liquid chromatograph was used for purification. The used chromatographic column was in a model No. Agela C18 (10 µm, 100Å, 50×250 mm), with a mobile phase consisting of a mobile phase A (0.05% TFA and 2% acetonitrile in water) and a mobile phase B (90% acetonitrile in water). The mobile phase had a flow rate of 25 mL/min, and the ultraviolet detection wavelength was 220 nm. The crude product solution was loaded on the chromatographic column to perform a gradient elution, and the corresponding purified components were collected and directly lyophilized to remove solvents, to obtain a pure product of trifluoroacetate of polypeptide in a fluffy state.

The pure product of trifluoroacetate of polypeptide was re-dissolved with water and acetonitrile, to which a large amount of anion exchange resin (in the form of an acetate radical) was added, the resulting mixture was stirred for 3 hours, and filtered, and then the ion exchange resin was rinsed with a water/acetonitrile mixed solvent, the filtrates were combined and lyophilized, to obtain a pure product of polypeptide acetic acid salt in a fluffy state.

The chemical structure of the synthesized lipopeptide was characterized by MALDI-TOF mass spectrometry, and the purity was determined by an analytical high performance liquid chromatography (Agela C18-4.6 × 250 mm, flow rate: 1 mL/min). The results showed that the synthesized lipopeptides had a purity of more than 95%.

### Example 2. Concept Verification of New Structure Lipopeptides

The present example identified the anti-HIV activity of the new structure lipopeptides with a HIV pseudovirus system and with lipopeptide LP-98 as a control of the new structure lipopeptides, and verified the superiority of the new structure lipopeptides in the concept.

Test lipopeptide: lipopeptide LP-101, lipopeptide LP-102, lipopeptide LP-103, lipopeptide LP-104, lipopeptide LP-105, lipopeptide LP-106, lipopeptide LP-107, lipopeptide LP-108, LP-109 or lipopeptide LP-98, as prepared in Example 1.

HIV-1_{JRFL} Env expression vectors and backbone plasmas (pSG3 ΔEnv) and TZM-bl cells were described in the following literature: Xue, J., Chong, H., Zhu, Y, Zhang, J., Tong, L., Lu, J., Chen, T., Cong, Z., Wei, Q., He, Y., 2022. Efficient treatment and pre-exposure prophylaxis in rhesus macaques by an HIV fusion-inhibitory lipopeptide. Cell 185, 131-144 e118. The HIV-1_{JRFL} Env expression vector and the backbone plasmas (pSG3 ΔEnv) were co-transfected into HEK293T cells, and the transfected cells were cultured in a cell culture incubator at 37 °C and 5% CO₂ for 48 hours, and then the supernatant was collected and filtered to collect the filtrate. The resulting filtrate was a virus solution containing HIV-1_{JRFL} pseudovirus particles (called as HIV-1_{JRFL} virus solution), which was preserved at -80 °C for later use.

The preparation method of the HIV-1_{NL4-3} virus solution (i.e., the virus solution containing HIV-1_{NL4-3} pseudovirus particle) was basically the same as that of the HIV-1_{JRFL} virus liquid, except that the HIV-1_{JRFL} Env expression vector was replaced with HIV-1_{NL4-3} Env expression vector. The HIV-1_{NL4-3} pseudovirus ("HIV-1_{NL4-3}" in a literature) was described in the following literature: Xue, J., Chong, H., Zhu, Y, Zhang, J., Tong, L., Lu, J., Chen, T., Cong, Z., Wei, Q., He, Y., 2022. Efficient treatment and pre-exposure prophylaxis in rhesus macaques by an HIV fusion-inhibitory lipopeptide. Cell 185, 131-144 e118.

Test virus solution: HIV-1_{NL4-3} virus solution or HIV-1_{JRFL} virus solution.
1. A test lipopeptide was dissolved in deionized water and then diluted with DMEM medium (using 3-fold dilution) to obtain a lipopeptide diluent. 9 dilutions were set for each test lipopeptide.
2. In a 96-well plate, the lipopeptide diluent (50 µL/well) was added to drug wells and the DMEM medium (50 µL/well) was added to control wells. Each well was set with three duplicate wells.
3. After step 2 was finished, a test virus solution (50 µL/well, a virus content of 100 TCID₅₀) was added to the 96-well plate and incubated at room temperature for 30 minutes.
4. After step 3 was finished, a TZM-b1 cell suspension (100 µL/well) was added to the 96-well plate and cultured for 48 hours in a cell culture incubator at 37 °C and 5% CO₂.
   Preparation method of the TZM-b1 cell suspension: TZM-b1 cells were re-suspended in a DMEM medium and DEAE-dextran was added thereto such that the cell concentration was 10×10⁴ cells/mL and the DEAE-dextran concentration was 15 µg/mL.
5. After step 4 was finished, the supernatant was discarded, and a cell lysate (30 µL/well) was added. The cell lysis was carried out for 15 minutes at room temperature, and then a luciferase assay substrate reagent was added. Relative luminescence units (RLU) were measured by a microplate luminometer, an inhibition rate curve was made, and the half-maximal inhibition concentration (IC₅₀) of the drug was calculated.

Cell lysate: Promega, Cat. No.: E1531.

Luciferase assay substrate reagent: Promega, Cat. No.: E1501.

The results were shown in FIG. 2.

Lipopeptide LP-101 was capable to potently inhibit infections of HIV-1_{NL4-3} pseudovirus and HIV-1_{JRFL} pseudovirus in TZM-b1 cells, with average IC₅₀ values of 0.46 pM and 2.26 pM, respectively, while lipopeptide LP-98 as a control inhibited HIV-1_{NL4-3} pseudovirus and HIV-1_{JRFL} pseudovirus with average IC₅₀ values of 1.07 pM and 2.58 pM, respectively.

Unexpectedly, lipopeptide LP-102, lipopeptide LP-103 and lipopeptide LP-104 show significantly reduced antiviral activity, especially lipopeptide LP-104 containing the full-length PBD sequence of 8 amino acids, which inhibited the HIV-1_{NL4-3} pseudovirus and HIV-1_{JRFL} pseudovirus with IC₅₀ values of 14.50 pM and 60.18 pM, respectively, and which, as compared to lipopeptide LP-101, was reduced by about 32-fold and 27-fold in the activity, respectively. The results showed that the lipopeptides containing two terminal amino acids E and I of PBD had the highest antiviral activity, and in contrast, the lipopeptides containing more amino acids of PBD had a significant reduction in the activity thereof, revealing the relationship between the structure and function of the lipopeptide inhibitors.

Lipopeptide LP-105 and lipopeptide LP-106 exhibited inhibitory activities similar to lipopeptide LP-101, indicating that the amino acid I inserted into the NHR hydrophobic pocket of the lipopeptides may be replaced by amino acid V or L without affecting the activity of the inhibitors.

Lipopeptide LP-107 and lipopeptide LP-108 also exhibited inhibitory activity similar to lipopeptide LP-101, indicating that different cholesterol modifications had no significant effect on the antiviral effects of the inhibitors.

### Example 3. Inhibitory Activity of New Structure Lipopeptides against HIV-Resistant strains

The HIV is prone to drug resistance, resulting in treatment failures, and this was a key problem to be solved in drug research and development. The present example used an HIV pseudovirus system to evaluate the inhibitory activities of the new-structure lipopeptides (with lipopeptide LP-101 and lipopeptide LP-108 as examples) against a plurality of HIV membrane fusion inhibitorresistant virus strains, and to confirm the druggability of the new structure lipopeptides, with lipopeptide LP-98 as a control of the new structure lipopeptides.

The T20-resistant NL4-3 mutant stain was an HIV-1_{NL4-3} mutant strain induced by T20, particularly containing the following mutations: I37T, V38A, V38M, Q40H, N43K, D36S/V38M, I37T/N43K, or V38A/N42T. The HIV-1_{NL4-3} virus (HIV-1_{NL4-3} WT in a literature) and each of the above mutant strains was described in the following literature (see Table 3 in the literature): Chong, H., Yao, X., Zhang, C., Cai, L., Cui, S., Wang, Y., He, Y., 2012. Biophysical property and broad anti-HIV activity of albuvirtide, a 3-maleimimidopropionic acid-modified peptide fusion inhibitor. PloS, 7, e32599.

The LP-40-induced drug-resistant NL4-3 strain was an HIV-1_{NL4-3} mutant strain induced by LP-40, particularly containing the following mutations: L33S, V38T, N42T, L33S/I37T, or L33S/V38A/N42T. The HIV-1_{NL4-3} virus (Pseudovirus NL4-3 in a literature) and each of the above mutant strains were described in the following literature (see Table 2 and Table 3 in the literature): Hu, Y, Yu, W., Geng, X., Zhu, Y, Chong, H., He, Y., 2022. In Vitro Selection and Characterization of HIV-1 Variants with increased Resistance to LP-40, Enfuvirtual-Based lipopeptide inhibitor. International journal of molecular science 23.

The LP-52-induced drug resistant SIV_{mac239} strain was an SIV_{mac239} mutant strain induced by LP-52, particularly containing the following mutations: V562A, V562M, V562A/E657G, V562M/E657G, V562A/S760G or V562M/S760G. The SIV_{mac239} virus ("Pseudovirus SIV_{mac239} WT" in a literature) and each of the above mutant strains were described in the following literature (see Table 2 in the literature): Yu, D., Xue, J., Wei, H., Cong, Z., Chen, T., Zhu, Y., Chong, H., Wei, Q., Qin, C., He, Y, 2020. Therapeutic Efficacy and Resistance Selection of a Lipopeptide Fusion Inhibitor in Simian Immunodeficiency Virus-Infected Rhesus Macaques. Journal of virology 94, e00384-00320.

The LP-52-induced drug-resistant NL4-3 strain was an HIV-1_{NL4-3} mutant strain induced by LP-52, particularly containing the following mutations: V547A/E646G, or V547M/E646G. The HIV-1_{NL4-3} virus ("Pseudovirus HIV-1_{NL4-3} WT" in a literature) and each of the above mutant strains were described in the following literature (see Table 2 in the literature): Yu, D., Xue, J., Wei, H., Cong, Z., Chen, T., Zhu, Y., Chong, H., Wei, Q., Qin, C., He, Y., 2020. Therapeutic Efficacy and Resistance Selection of a Lipopeptide Fusion Inhibitor in Simian Immunodeficiency Virus-Infected Rhesus Macaques. Journal of virology 94, e00384-00320.

The SC29EK-induced drug-resistant NL4-3 strain was an HIV-1_{NL4-3} mutant strain induced by SC29EK, particularly containing the following mutations: E49A, N43K/E49A, Q39R/N43K/N126K or N43K/E49A/N126K. The HIV-1_{NL4-3} virus (HIV-1_{NL4-3} Wild type in a literature) and each of the above mutant strains were described in the following literature (see Table 1 in the literature): Wu, X., Liu, Z., Ding, X., Yu, D., Wei, H., Qin, B., Zhu, Y., Chong, H., Cui, S., He, Y., 2018. Mechanism of HIV-1 Resistance to an Electronically Constrained alpha-Helical Peptide Membrane Fusion Inhibitor. Journal of virology 92, e02044-02017.

The SC22EK-induced drug-resistant NL4-3 strain was an HIV-1_{NL4-3} mutant strain induced by SC22EK, particularly containing the following mutations: E49K, N126K, or E49K/N126K. The HIV-1_{NL4-3} virus (HIV-1_{NL4-3} virus Wild type in a literature) and each of the above mutants were described in the following literature (see Table 1 in the literature): Su, Y., Chong, H., Qiu, Z., Xiong, S., He, Y., 2015a. Mechanism of HIV-1 Resistance to Short-Peptide Fusion Inhibitors Targeting the Gp41 Pocket. Journal of virology 89, 5801-5811.

The MTSC22-induced drug-resistant NL4-3 strain was an HIV-1_{NL4-3} mutant strain induced by MTSC22, particularly containing the following mutations: L57R, E136G, or L57R/E136G. The HIV-1_{NL4-3} virus (HIV-1_{NL4-3} Wild type in the literature) and each of the above mutant strains was described in the following literature (see Table 1 in the literature): Su, Y, Chong, H., Xiong, S., Qiao, Y, Qiu, Z., He, Y., 2015b. Genetic Pathway of HIV-1 Resistance to Novel Fusion Inhibitors Targeting the Gp41 Pocket. Journal of virology 89, 12467-12479.

The SFT-induced drug-resistant NL4-3 strain was an HIV-1_{NL4-3} mutant strain induced by SFT, particularly containing the following mutation: Q52R. The HIV-1_{NL4-3} virus (HIV-1_{NL4-3} virus WT in a literature) and the above mutant strain were described in the following literature (see Table 1 in the literature): Yu, D., Ding, X., Liu, Z., Wu, X., Zhu, Y., Wei, H., Chong, H., Cui, S., He, Y., 2018. Molecular mechanism of HIV-1 resistance to sifuvirtide, a clinical trial-approved membrane fusion inhibitor. The Journal of biological chemistry 293, 12703-12718.

Test virus solution: virus solution of each T20-resistant NL4-3 mutant strain, virus solution of each LP-40-induced drug-resistant NL4-3 mutant strain, virus solution of each LP-52-induced drug-resistant SIV_{mac239} mutant strain, virus solution of each LP-52-induced drug-resistant NL4-3 mutant strain, virus solution of each SC29EK-induce drug-resistant NL4-3 mutant strain, virus solution of each SC22EK-induced drug-resistant NL4-3 mutant strain, virus solution of each MTSC22-induced drug-resistant NL4-3 mutant strain, or virus solution of SFT-induced drug-resistant NL4-3 mutant strain.

Test lipopeptide: lipopeptide LP-101, lipopeptide LP-108 or lipopeptide LP-98, as prepared in Example 1.

The method was the same as that in Example 2.

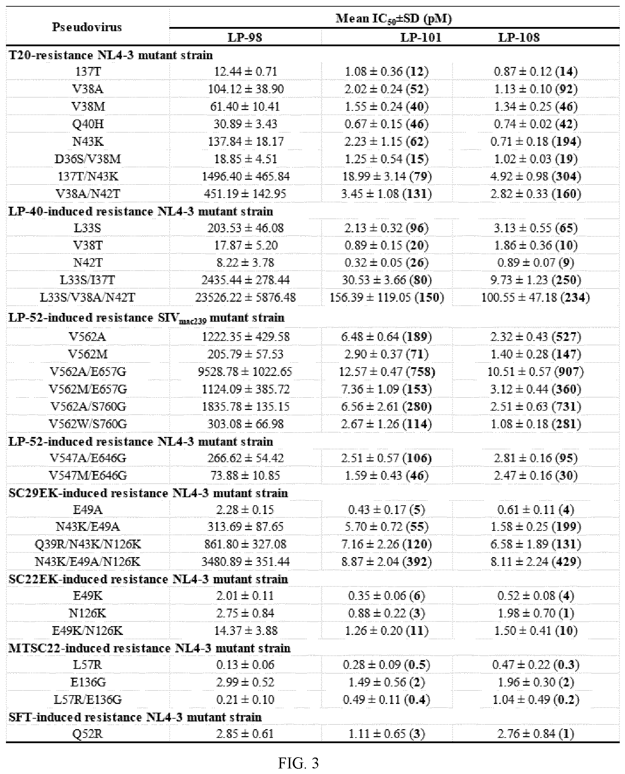

The results were shown in FIG. 3.

The inhibitory activities of lipopeptide LP-101 against various T20-resistant NL4-3 mutant strains were increased by 12 folds to 131 folds as compared to lipopeptide LP-98, and those of lipopeptide LP-108 were increased by 14 folds to 304 folds as compared to lipopeptide LP-98. The inhibitory activities of lipopeptide LP-101 against various LP-40-induced drug-resistant NL4-3 strains were increased by 20 folds to 150 folds as compared to lipopeptide LP-98, and those of lipopeptide LP-108 were increased by 9 folds to 250 folds as compared to lipopeptide LP-98. The inhibitory activities of lipopeptide LP-101 against various LP-52-induced drug-resistant SIVmac239 strains were increased by 71 folds to 758 folds as compared with lipopeptide LP-98, and those of lipopeptide LP-108 were increased by 147 folds to 907 folds as compared with lipopeptide LP-98. The inhibitory activities of lipopeptide LP-101 against two LP-52-induced drug-resistant NL4-3 strains were increased by 106 folds or 46 folds as compared to LP-98, and those of lipopeptide LP-108 were increased by 95 folds or 30 folds as compared to lipopeptide LP-98. The inhibitory activities of lipopeptide LP-101 against various SC29EK-induced drug-resistant NL4-3 strains were increased by 5 folds to 392 folds as compared to lipopeptide LP-98, and those of lipopeptide LP-108 were increased by 4 folds to 429 folds as compared to lipopeptide LP-98. The inhibitory activities of lipopeptide LP-101 against various SC22EK-induced drug-resistant NL4-3 strains were increased by 3 folds to 11 folds as compared to lipopeptide LP-98, and those of lipopeptide LP-108 were increased by 1 fold to 10 folds as compared to lipopeptide LP-98. Regarding the inhibitory activities against various MTSC22-induced drug-resistant NL4-3 strains, the IC₅₀ values of lipopeptide LP-98 were 0.4 folds to 2 folds as those of lipopeptide LP-101, and 0.2 folds to 2 folds as those of lipopeptide LP-108. The inhibitory activity of lipopeptide LP-101 against the SFT-induced drug-resistant NL4-3 strain was increased by 3 folds as compared to lipopeptide LP-98, while lipopeptide LP-108 had similar activity as compared to lipopeptide LP-98.

Since T20, LP-40, and LP-52 each did not have PBD amino acids, but SC29EK, SC22EK, MTSC22, and SFT contained a complete PBD sequence, especially the MTSC22 further contained an additional N-terminal "M-T hook" structure, the above-described contents further deeply revealed the relationship between structure and function of lipopeptide LP-101, lipopeptide LP-108, and lipopeptide LP-98 as inhibitors.

### Example 4. Comparisons of Inhibitory Activities of Lipopeptides against HIV-2 and SIV

Test virus solution: HIV-2_{ROD} virus solution, HIV-2_{ST} virus solution, SIV_{mac239} virus solution or SIV_{PBJ} virus solution.

Both the HIV-2_{ROD} virus and the HIV-2_{ST} virus were infectious HIV-2 viruses (Virus type: Replicative). Both the SIV_{mac239} virus and the SIV_{PBJ} virus were SIV pseudoviruses (Virus type: Pseudotype). The HIV-2_{ROD} virus, the HIV-2_{ST} virus, the SIV_{mac239} virus and the SIV_{PBJ} virus each were described in the following literature (see Table S1 in the literature): Xue, J., Chong, H., Zhu, Y., Zhang, J., Tong, L., Lu, J., Chen, T., Cong, Z., Wei, Q., He, Y, 2022. Efficient treatment and pre-exposure prophylaxis in rhesus macaques by an HIV fusion-inhibitory lipopeptide. Cell 185, 131-144 e118.

Test lipopeptide: lipopeptide LP-101, lipopeptide LP-108 or lipopeptide LP-98, as prepared in Example 1.

The method was the same as that in Example 2.

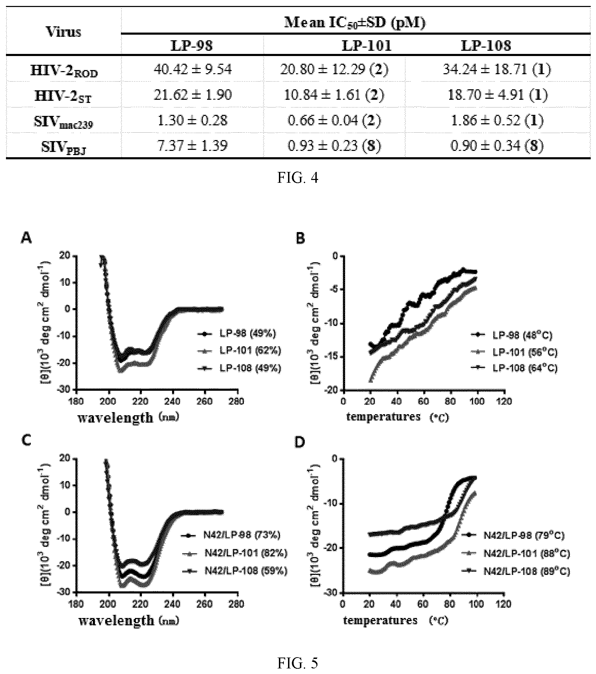

The results were shown in FIG. 4. As compared with lipopeptide LP-98, the inhibitory activities of lipopeptide LP-101 against HIV-2_{ROD} virus and HIV-2_{ST} virus were increased by 2 folds and 2 folds, respectively, and the inhibitory activities against SIV_{mac239} virus and SIV_{PBJ} virus were increased by 2 folds and 8 folds, respectively. The inhibitory activities of lipopeptide LP-108 against HIV-2_{ROD} virus, HIV-2_{ST} virus and SIV_{mac239} virus were comparable to those of lipopeptide LP-98, and its inhibitory activity against SIV_{PBJ} virus was increased by 8 folds as compared with lipopeptide LP-98. Therefore, the lipopeptides of the present application had potent inhibitory activities against HIV-2 and SIV, further reflecting their broad-spectrum antiviral effect.

### Example 5. Helix Structural Characteristics of Lipopeptides and Binding Stability Analyses thereof

In order to analyze the structural features of the lipopeptides of the present application and investigate their mechanism of action, a circular dichroism (CD) technique was used to determine the secondary structure (α-helix) and helix stability (Tm value) of the lipopeptide LP-101 and lipopeptide LP-108 prepared in the examples of the present application, as well as their complexes with a target sequence. The lipopeptide LP-98 was used as a control.

### I. Experimental materials and methods

NHR polypeptides: polypeptide N42, which was correspondent to the NHR sequence of gp41 fusion proteins and used as a simulated target of lipopeitide inhibitors, was synthesized in the laboratory and commonly used, and see the literature (Xue et al., 2022) published by the inventors. The polypeptide N42 is: Ac-STMGAASMTLTVQARQLLSGIVQQQNNLLRAIEAQQHLLQLT-NH₂, with the amino acid sequence as forth in SEQ ID NO: 11.

CD determination method: lipopeptide LP-101, lipopeptide LP-108, lipopeptide LP-98, a mixture of N42 polypeptide and lipopeptide LP-101 (N42/LP-101), a mixture of N42 polypeptide and lipopeptide LP-108 (N42/LP-108), and a mixture of N42 polypeptide and lipopeptide LP-98 (N42/LP-98) were dissolved in in a phosphate buffered saline (PBS) with a pH of 7.2, respectively, to obtain solutions having both the final concentrations of the lipopeptides and N42 polypeptide of 10 µM. Each solution was placed in a water bath at 37 °C for 30 minutes and then transferred to a corresponding cuvette. A JASCO spectropolarimeter (Model J-815) was used to scan changes of the molar ellipticity [θ]λ of the solution in the wavelength range of 195-270 nm. the typical α-helical structure could exhibit maximum negative peaks at 208 nm and 222 nm. The PBS blank control was subtracted to correct the spectrum values. During the calculations, the peak value of - 33000 degree.cm².dmol⁻¹ was use as the standard of 100% α-helix content, the percentages of the α-helix contents of the polypeptides were calculated according to the molar ellipticity of the solution at 222 nm. Subsequently, the solution was added to a corresponding cuvette for detecting thermal stability, and the CD temperature control module was adjusted to scan the polypeptide solution at a speed of 2 °C/min to detect the temperature-dependent changes in [θ]222 at the range of 20-98 °C. The melting curve was smoothed and the midpoint temperature value (Tm) of the thermal dissociation transition was calculated using Origin software, to reflect the degree of helix thermal stability.

### II. Experimental Results and Analysis

The results were shown in FIG. 5.

The α-helix contents of lipopeptide LP-98, lipopeptide LP-101, and lipopeptide LP-108 alone were 49%, 62% and 49%, respectively (FIG. 5A), indicating that lipopeptide LP-101 had a high α-helicity. The Tm values of lipopeptide LP-98, lipopeptide LP-101 and lipopeptide LP-108 alone were 48 °C, 56 °C and 64 °C, respectively (FIG. 5B), indicating that for the helix stabilities of the three lipopetides, lipopeptide LP-108 was highest, lipopeptide LP-101 was secondary, and lipopeptide LP-98 was relatively lower.

The α-helix contents of the complexes formed by lipopeptide LP-98, lipopeptide LP-101 and lipopeptide LP-108 with N42 polypeptide were 73%, 82% and 59%, respectively (FIG. 5C), and their Tm values were 79 °C, 88 °C and 89 °C, respectively (FIG. 5D). It was indicated that the helicity of the N42/LP-101 complex was relatively higher, and the helicity of the N42/LP-108 complex was relatively lower, whereas the two complexes had close helix stabilities, significantly higher than the helix stability of the N42/LP-98 complex.

The present application has been described in detail above. In the case of not departing from the spirit and scope of the present application and in no need of conducting unnecessary experiments, one of ordinary skill in the art could implement the present application in a broad scope at equivalent parameters, concentrations and conditions. While the present application provides special examples, it will be appreciated that the present application may be further modified. In general, according to the principles of the present application, the present application is intended to cover any alterations, uses, or improvements to the present application, including changes made with known routine techniques in the art with departing from the scope as disclosed in the present application. According to the scope of the claims attached below, some basic features may be applied.

## Claims

1. A lipopeptide, a pharmaceutically acceptable salt thereof, a solvate thereof, a hydrate thereof or a derivative thereof, **characterized in that**:
the lipopeptide is a compound represented by Formula I;
Formula I: X₁-polypeptide P1-X₂;
the polypeptide P1 is the following (a1) or (a2) or (a3):
(a1) a polypeptide with an amino acid sequence "EXEELEKKIEELLKKAEEQQKKNEEELKKLEK";
(a2) a polypeptide with antiviral activity obtained by substitution and/or deletion and/or addition of amino acid residue on the basis of (a1);
(a3) a polypeptide with a sequence identity of at least 60%, 65%, 70%, 75%, 80%, 85%, 90%, 93%, 94%, 95%, 96%, 97%, 98%, or 99% as compared with (a1) and having antiviral activity;
a terminal amino acid residue of the polypeptide P1 is modified with a lipophilic compound group;
X₁ is an amino-terminal protecting group of the polypeptide P1;
X₂ is a carboxyl-terminal protecting group of the polypeptide P1.

2. The lipopeptide, a pharmaceutically acceptable salt thereof, a solvate thereof, a hydrate thereof or a derivative thereof according to claim 1, **characterized in that**:
in the polypeptide P1, X is isoleucine, valine or leucine.

3. A lipopeptide, a pharmaceutically acceptable salt thereof, a solvate thereof, a hydrate thereof or a derivative thereof, **characterized in that**:
the lipopeptide is a compound represented by Formula II;
Formula II: X₃-polypeptide P2-X₄;
the polypeptide P2 is the following (b1) or (b2) or (b3):
(b1) a polypeptide with an amino acid sequence "EXEELEKKIEELLKKAEEQQKKNEEELKKLEKX";
(b2) a polypeptide with antiviral activity obtained by substitution and/or deletion and/or addition of amino acid residue on the basis of (b1);
(b3) a polypeptide with a sequence identity of at least 60%, 65%, 70%, 75%, 80%, 85%, 90%, 93%, 94%, 95%, 96%, 97%, 98%, or 99% as compared with (b1) and having antiviral activity;
a terminal amino acid residue of the polypeptide P2 is modified with a lipophilic compound group;
X₃ is an amino-terminal protecting group of the polypeptide P2;
X₄ is a carboxyl-terminal protecting group of the polypeptide P2.

4. The lipopeptide, a pharmaceutically acceptable salt thereof, a solvate thereof, a hydrate thereof or a derivative thereof according to claim 3, **characterized in that**:
in the polypeptide P2, "X" as the 2^{nd} amino acid residue is isoleucine, valine, or leucine, and "X" as the 33^{rd} amino acid residue is lysine or cysteine.

5. The lipopeptide, a pharmaceutically acceptable salt thereof, a solvate thereof, a hydrate thereof or a derivative thereof according to any one of claims 1 to 4, **characterized in that**:
the lipophilic compound is cholesterol, a cholesterol derivative (such as cholesteryl hemisuccinate, 2-cholesteryl acetic acid, 2-cholesteryl propionic acid, 3-cholesteryl propionic acid, 2-cholesteryl butyric acid, 2-cholesteryl isobutyric acid, 3-cholesteryl butyric acid, 3-cholesteryl isobutyric acid, 4-cholesteryl butyric acid, 2-cholesteryl valeric acid, 2-cholesteryl isovaleric acid, 3-cholesteryl valeric acid, 5-cholesteryl valeric acid, 2-cholesteryl caproic acid, 6-cholesteryl caproic acid, 2-cholesteryl enanthic acid, 7-cholesteryl enanthic acid, 2-cholesteryl caprylic acid, 8-cholesteryl caprylic acid, and cholesteryl bromoacetate), a fatty acid containing 8 to 20 carbon atoms (such as octadecanoic acid or palmitic acid), dihydrosphingosine (DHS) or vitamin E;
preferably, the lipophilic compound is cholesterol;
preferably, the lipophilic compound is cholesteryl hemisuccinate;
preferably, the lipophilic compound is cholesteryl bromoacetate;
preferably, the lipophilic compound is stearic acid.

6. The lipopeptide, a pharmaceutically acceptable salt thereof, a solvate thereof, a hydrate thereof or a derivative thereof according to any one of claims 1 to 5, **characterized in that**:
the X₁ is any one selected from the group consisting of acetyl, amino, maleoyl, succinyl, tert-butoxycarbonyl, benzyloxy, another hydrophobic group and macromolecular carrier group;
the X₂ is any one selected from the group consisting of amino, carboxyl, hydroxyl, amido, tert-butoxycarbonyl, another hydrophobic and macromolecular carrier group;
the X₃ is any one selected from the group consisting of acetyl, amino, maleoyl, succinyl, tert-butoxycarbonyl, benzyloxy, another hydrophobic group and macromolecular carrier group;
the X₄ is any one selected from the group consisting of amino, carboxyl, hydroxyl, amido, tert-butoxycarbonyl, another hydrophobic group and macromolecular carrier group.

7. The lipopeptide, a pharmaceutically acceptable salt thereof, a solvate thereof, a hydrate thereof or a derivative thereof according to any one of claims 1 to 6, **characterized in that**:
the polypeptide P1 is the following (c1) or (c2) or (c3) or (c4) or (c5):
(c1) a polypeptide with a sequence as set forth in SEQ ID NO: 1 or SEQ ID NO: 9 in the sequence listing;
(c2) a polypeptide with a sequence as set forth in SEQ ID NO: 5 in the sequence listing;
(c3) a polypeptide with a sequence as set forth in SEQ ID NO: 6 in the sequence listing;
(c4) a polypeptide with antiviral activity obtained by substitution and/or deletion and/or addition of amino acid residue on the basis of the polypeptide of any one of (c1) to (c3);
(c5) a polypeptide with a sequence identity of at least 60%, 65%, 70%, 75%, 80%, 85%, 90%, 93%, 94%, 95%, 96%, 97%, 98% or 99% as compared with the polypeptides of any one of (c1) to (c3) and having antiviral activity;
the polypeptide P2 is the following (d1) or (d2) or (d3) or (d4):
(d1) a polypeptide with a sequence as set forth in SEQ ID NO: 7 in the sequence listing;
(d2) a polypeptide with a sequence as set forth in SEQ ID NO: 8 in the sequence listing;
(d3) a polypeptide with antiviral activity obtained by substitution and/or deletion and/or addition of amino acid residue on the basis of the polypeptide of any one of (d1) to (d2);
(d4) a polypeptide with a sequence identity of at least 60%, 65%, 70%, 75%, 80%, 85%, 90%, 93%, 94%, 95%, 96%, 97%, 98% or 99% as compared with the polypeptide of any one of (d1) to (d2) and having antiviral activity.

8. A polypeptide, **characterized in that**:
the polypeptide is the following (a1) or (a2) or (a3):
(a1) a polypeptide with an amino acid sequence "EXEELEKKIEELLKKAEEQQKKNEEELKKLEK", wherein X is isoleucine, valine or leucine;
(a2) a polypeptide with antiviral activity obtained by substitution and/or deletion and/or addition of amino acid residue on the basis of (a1);
(a3) a polypeptide with a sequence identity of at least 60%, 65%, 70%, 75%, 80%, 85%, 90%, 93%, 94%, 95%, 96%, 97%, 98%, or 99% as compared with (a1) and having antiviral activity; or the polypeptide is the following (b1) or (b2) or (b3):
(b1) a polypeptide with an amino acid sequence "EXEELEKKIEELLKKAEEQQKKNEEELKKLEKX", wherein "X" as the 2^{nd} amino acid residue is isoleucine, valine or leucine, and "X" as the 33^{rd} amino acid residue is lysine or cysteine;
(b2) a polypeptide with antiviral activity obtained by substitution and/or deletion and/or addition of amino acid residue on the basis of (b1);
(b3) a polypeptide with a sequence identity of 60%, 65%, 70%, 75%, 80%, 85%, 90%, 93%, 94%, 95%, 96%, 97%, 98%, or 99% as compared with (b1) and having antiviral activity;
preferably, the polypeptide is the following (c1) or (c2) or (c3) or (c4) or (c5):
(c1) a polypeptide with a sequence as set forth in SEQ ID NO: 1 or SEQ ID NO: 9 in the sequence listing;
(c2) a polypeptide with a sequence as set forth in SEQ ID NO: 5 in the sequence listing;
(c3) a polypeptide with a sequence as set forth in SEQ ID NO: 6 in the sequence listing;
(c4) a polypeptide having an antiviral activity, obtained by substitution and/or deletion and/or addition of amino acid residue on the basis of the polypeptide of any one of (c1) to (c3);
(c5) a polypeptide with a sequence identity of at least 60%, 65%, 70%, 75%, 80%, 85%, 90%, 93%, 94%, 95%, 96%, 97%, 98% or 99% as compared with the polypeptide of any one of (c1) to (c3) and having antiviral activity; or
the polypeptide is the following (d1) or (d2) or (d3) or (d4):
(d1) a polypeptide with a sequence as set forth in SEQ ID NO: 7 in the sequence listing;
(d2) a polypeptide with a sequence as set forth in SEQ ID NO: 8 in the sequence listing;
(d3) a polypeptide with antiviral activity obtained by substitution and/or deletion and/or addition of amino acid residue on the basis of the polypeptide of any one of (d1) to (d2);
(d4) a polypeptide with a sequence identity of at least 60%, 65%, 70%, 75%, 80%, 85%, 90%, 93%, 94%, 95%, 96%, 97%, 98% or 99% as compared with the polypeptide of any one of (d1) to (d2) and having antiviral activity.

9. A conjugate comprising the polypeptide according to claim 8 and a modification moiety;
for example, wherein the modification moiety is linked to the N-terminus or C-terminus of the polypeptide, optionally through a linker;
for example, wherein the modification moiety is a terminal protecting group.

10. An isolated nucleic acid, which encodes the polypeptide according to claim 8.

11. A vector comprising the isolated nucleic acid according to claim 10.

12. A host cell comprising the isolated nucleic acid according to claim 10 and/or the vector according to claim 11.

13. A multimer, which is the following (e1) or (e2) or (e3) or (e4) or (e5):
(e1) a multimer formed from the lipopeptide according to any one of claims 1 to 7;
(e2) a multimer formed from the pharmaceutically acceptable salt according to any one of claims 1 to 7;
(e3) a multimer formed from the derivative according to any one of claims 1 to 7;
(e4) a multimer formed from the hydrate according to any one of claims 1 to 7;
(e5) a multimer formed from the solvate according to any one of claims 1 to 7; or
the multimer is a multimer formed with the polypeptide according to claim 8.

14. A composition, comprising the lipopeptide, a pharmaceutically acceptable salt thereof, a solvate thereof, a hydrate thereof or a derivative thereof according to any one of claims 1 to 7, or the polypeptide according to claim 8, or the conjugate according to claim 9, or the isolated nucleic acid according to claim 10, or the vector according to claim 11, or the host cell according to claim 12, or the multimer according to claim 13.

15. A pharmaceutical composition, comprising the lipopeptide, a pharmaceutically acceptable salt thereof, a solvate thereof, a hydrate thereof or a derivative thereof according to any one of claims 1 to 7, or the polypeptide according to claim 8, optionally further comprising a pharmaceutically acceptable carrier,
preferably, the lipopeptide, a pharmaceutically acceptable salt thereof, a solvate thereof, a hydrate thereof or a derivative thereof or the polypeptide is present in an amount effective for treating a viral infection or a disease caused by viral infection;
preferably, the viral infection is an infection caused by a virus selected from the group consisting of HIV, SIV, and resistant strain thereof;
preferably, the HIV is HIV-1 and/or HIV-2;
preferably, the HIV is a T20 resistant virus;
preferably, the HIV is an LP-98 resistant virus;
preferably, the viral infection is an infection caused by a virus selected from the group consisting of a T20 resistant virus, an LP-40 resistant virus, an LP-52 resistant virus, an SC29EK resistant virus, an SC22EK resistant virus, an MTSC22 resistant virus, and an SFT resistant virus;
preferably, the disease caused by viral infection is AIDS.

16. Use of the lipopeptide, a pharmaceutically acceptable salt thereof, a solvate thereof, a hydrate thereof or a derivative thereof according to any one of claims 1 to 7 or the polypeptide according to claim 8 or the multimer according to claim 13, which is the following (f1) or (f2) or (f3) or (f4):
(f1) use in the manufacture of a viral membrane fusion inhibitor;
(f2) use in the manufacture of a medicament for preventing and/or treating a disease caused by viral infection;
(f3) use as a viral membrane fusion inhibitor;
(f4) use in prevention and/or treatment of a disease caused by viral infection;
preferably, the viral infection is an infection caused by a virus selected from the group consisting of HIV, SIV, and resistant strain thereof;
preferably, the HIV is HIV-1 and/or HIV-2;
preferably, the HIV is a T20 resistant virus;
preferably, the HIV is an LP-98 resistant virus;
preferably, the viral infection is an infection caused by a virus selected from the group consisting of a T20 resistant virus, an LP-40 resistant virus, an LP-52 resistant virus, an SC29EK resistant virus, an SC22EK resistant virus, an MTSC22 resistant virus, and an SFT resistant virus;
preferably, the disease caused by viral infection is AIDS.

17. A product comprising the lipopeptide or a pharmaceutically acceptable salt thereof or a solvate thereof or a hydrate thereof or a derivative thereof according to any one of claims 1 to 7 or the polypeptide according to claim 8 or the multimer according to claim 13, wherein the product having the following functions (g1) or (g2):
(g1) function as a viral membrane fusion inhibitor;
(g2) function of preventing and/or treating a disease caused by viral infection;
preferably, the viral infection is an infection caused by a virus selected from the group consisting of HIV, SIV, and resistant strain thereof;
preferably, the HIV is HIV-1 and/or HIV-2;
preferably, the HIV is a T20 resistant virus;
preferably, the HIV is an LP-98 resistant virus;
preferably, the viral infection is an infection caused by a virus selected from the group consisting of a T20 resistant virus, an LP-40 resistant virus, an LP-52 resistant virus, an SC29EK resistant virus, an SC22EK resistant virus, an MTSC22 resistant virus, and an SFT resistant virus;
preferably, the disease caused by viral infection is AIDS.

18. A method for treating or/and preventing a viral infection in an animal, comprising administering an effective amount of the lipopeptide or a pharmaceutically acceptable salt thereof or solvate thereof or a hydrate thereof or a derivative thereof according to any one of claims 1 to 7 or the polypeptide according to claim 8 or the multimer according to claim 13 or the pharmaceutical composition according to claim 15 to a subject animal;
preferably, the viral infection is an infection caused by a virus selected from the group consisting of HIV, SIV, and resistant strain thereof;
preferably, the HIV is HIV-1 and/or HIV-2;
preferably, the HIV is a T20 resistant virus;
preferably, the HIV is an LP-98 resistant virus;
preferably, the viral infection is an infection selected from the group consisting of a T20 resistant virus, an LP-40 resistant virus, an LP-52 resistant virus, an SC29EK resistant virus, an SC22EK resistant virus, an MTSC22 resistant virus, and an SFT resistant virus;
preferably, the animal is a mammal, e.g., a human.

19. A method for treating or/and preventing a disease caused by viral infection, comprising administering an effective amount of the lipopeptide or a pharmaceutically acceptable salts thereof or a solvate thereof or a hydrate thereof or a derivative thereof according to any one of claims 1 to 7 or the polypeptide according to claim 8 or the multimer according to claim 13 or the pharmaceutical composition according to claim 15 to a subject in need thereof;
preferably, the viral infection is an infection caused by a virus selected from the group consisting of HIV, SIV, and resistant strain thereof;
preferably, the HIV is HIV-1 and/or HIV-2;
preferably, the HIV is a T20 resistant virus;
preferably, the HIV is an LP-98 resistant virus;
preferably, the viral infection is an infection caused by a virus selected from the group consisting of a T20 resistant virus, an LP-40 resistant virus, an LP-52 resistant virus, an SC29EK resistant virus, an SC22EK resistant virus, an MTSC22 resistant virus, and an SFT resistant virus;
preferably, the disease caused by viral infection is AIDS;
preferably, the subject is a mammal, e.g., a human.
